# EUROPEAN PATENT SPECIFICATION

(11) **EP 2 167 503 B1**
(45) Date of publication and mention of the grant of the patent: **24.10.2012**
(21) Application number: 08773955.3
(22) Date of filing: 10.07.2008
(51) Int. Cl.: C07D 451/02, C07D 451/04, C07D 451/06, C07D 519/00, A01N 43/90, A01P 3/00, A01P 7/00, A01P 9/00, A01P 5/00

(54) **TROPANE DERIVATIVES USEFUL AS PESTICIDES**
ALS PESTIZIDE GEEIGNETE TROPANDERIVATE
DÉRIVÉS DE TROPANE UTILES COMME PESTICIDES

(30) Priority: 12.07.2007 GB 0713602
(43) Date of publication of application: 31.03.2010
(73) Proprietor: Syngenta Participations AG, 4058 Basel (CH); Syngenta Limited, Guildford Surrey GU2 7YH (GB)
(72) Inventor: SELLES, Patrice, CH-4332 Stein (CH); CLARKE, Eric, Daniel, Berkshire RG42 6EY (GB); ELLIOTT, Alison, Clare, Berkshire RG42 6EY (GB); FAWKE, Delphine, Raymonde, Suzanne, Berkshire RG42 6EY (GB); HUETER, Ottmar, Franz, CH-4332 Basel (CH); MUELLER, Urs, CH-4142 Münchenstein (CH); RENOLD, Peter, CH-4332 Stein (CH); TARGETT, Sarah, Margaret, Berkshire RG42 6EY (GB); WHITTINGHAM, William, Guy, Berkshire RG42 6EY (GB)
(74) Representative: Hölscher, Ingo
(86) International application number: PCT/EP2008/005633
(87) International publication number: WO 2009/007115

(56) References cited:
- EP-A- 1 431 299
- EP-A- 1 731 518
- EP-A- 1 932 844
- WO-A-96/37494
- WO-A-2006/100037

## Description

The present invention relates to tropane derivatives, to processes for preparing them, to pesticidal, in particular fungicidal, insecticidal, acaricidal, molluscicidal and nematicidal compositions comprising them and to methods of applying them to a plant to combat and control fungi, insect, acarine, mollusc and nematode pests. The present invention also relates to the use of a compound of formula (I) in the manufacture of a medicament for controlling pests on domestic animals and productive livestock.

Tropane derivatives with insecticidal activity are disclosed, for example, in WO-A-2006/100037 and WO 96/37494. WO 01/44249 and EP-A-435381 describe tropane derivatives with pharmaceutical activity.

Novel tropane derivatives have now been found which have fungicidal and insecticidal, acaricidal, molluscicidal and nematicidal properties.

The present invention therefore provides compounds of formula (I) 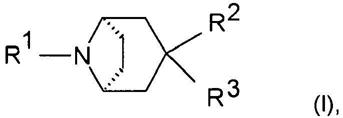 wherein
R¹ is pyrimidinyl, quinazolinyl or thienopyrimidinyl, where these rings are optionally substituted by C₁₋₄alkyl, C₁₋₄alkoxy, C₁₋₄alkylthio, C₁₋₄haloalkyl, halogen, cyano or C₁₋₄alkylcarbonyl,
R³ together with R² form a radical of the formula =CH-R⁴, wherein R⁴ is phenyl, which is optionally substituted by C₁₋₄ -alkyl, C₁₋₄alkoxy or halogen, or R⁴ is allyloxymethyl or phenoxymethyl, which is optionally substituted by C₁₋₄alkyl, C₁₋₄alkoxy or halogen, or R⁴ is pyridyloxymethyl, which is optionally substituted by C₁₋₄alkyl, or R⁴ is pyrimidinyloxymethyl, which is optionally substituted by C₁₋₄alkyl, or R⁴ is C₁₋₆alkylcarbonyl, C₁₋₄alkyl-aminocarbonyl, benzylaminocarbonyl, phenylaminocarbonyl, which is optionally substituted by halogen, pyridylaminocarbonyl, which is optionally substituted by C₁₋₄alkyl, or pyridazineaminocarbonyl, which is optionally substituted by C₁₋₄alkyl or halogen, or
R³ together with R² form a radical of the formula =N-O-(CH₂)ₙ-R⁵, where n is 0 or 1, and R⁵ is C₁₋₄alkyl, C₁₋₄alkoxy, C₂₋₄alkenyl or C₂₋₄alkynyl, or R⁵ is phenyl, pyridyl or oxadiazoly, where these rings are optionally substituted by C₁₋₄alkyl, C₁₋₄alkoxy, C₁₋₄haloalkyl, halogen or -SO₂C₁₋₄alkyl,
or salts or N-oxides thereof.

The compounds of formula (I) may exist in different geometric or optical isomers or tautomeric forms. This invention covers all such isomers and tautomers and mixtures thereof in all proportions as well as isotopic forms such as deuterated compounds.

Each alkyl moiety either alone or as part of a larger group (such as alkoxy, alkylcarbonyl, is a straight or branched chain and is, for example, methyl, ethyl, n-propyl, n-butyl, n-pentyl, n-hexyl, iso-propyl, n-butyl, sec-butyl, iso-butyl, tert-butyl or neo-pentyl. The alkyl groups are suitably C₁-C₄ alkyl groups.

Halogen is fluorine, chlorine, bromine or iodine.

Halogen-substituted carbon-containing groups and compounds, such as, for example, halogen-substituted alkyl, can be partially halogenated or perhalogenated, where in the case of polyhalogenation the halogen substituents can be identical or different. Examples of haloalkyl are methyl which is mono- to trisubstituted by fluorine, chlorine and/or bromine, such as CHF₂ or CF₃; ethyl which is mono- to pentasubstituted by fluorine, chlorine and/or bromine, such as CH₂CF₃, CF₂CF₃, CF₂CCl₃, CF₂CHCl₂, CF₂CHF₂, CF₂CFCl₂, CF₂CHBr₂, CF₂CHClF, CF₂CHBrF or CClFCHClF; propyl or isopropyl which is mono- to heptasubstituted by fluorine, chlorine and/or bromine, such as CH₂CHBrCH₂Br, CF₂CHFCF₃, CH₂CF₂CF₃, CF(CF₃)₂ or CH(CF₃)₂; butyl or one of its isomers, mono- to nonasubstituted by fluorine, chlorine and/or bromine, such as CF(CF₃)CHFCF₃ or CH₂(CF₂)₂CF₃.

Most preferably, R¹ is thienopyrimidinyl, which is optionally substituted by C₁₋₄alkyl, which is optionally further substituted, C₁₋₄alkoxy, C₁₋₄alkylthio, C₁₋₄haloalkyl, halogen, cyano or C₁₋₄alkylcarbonyl.

Preferably, R⁴ is phenyl, which is optionally substituted by C₁₋₄ -alkyl, C₁₋₄alkoxy or halogen.

Preferrably, R⁵ is phenyl, which is optionally substituted by C₁₋₄alkyl, C₁₋₄alkoxy, C₁₋₄haloalkyl or halogen, or R⁵ is oxadiazolyl, which is optionally substituted by C₁₋₄alkyl.

The compounds of the invention may be made in a variety of ways. In the section that follows, R¹, R², R³, R⁴ and R⁵ and n are as defined for formula (I) above unless otherwise stated.

Compounds of the invention can be prepared from readily available starting material. The compound of general formula (I) can be prepared by state of the art reaction presented below but also other methods described in the literature. The tropane ring, where PG is a protective group such as benzyl, Methyl, BOC, can be prepared by readily available starting material following the Robinson (J. Chem. Soc., 1917, 111, 762) or modified Robinson synthesis (Synlett, 2004, 1, 143-145) exemplified in the following scheme. 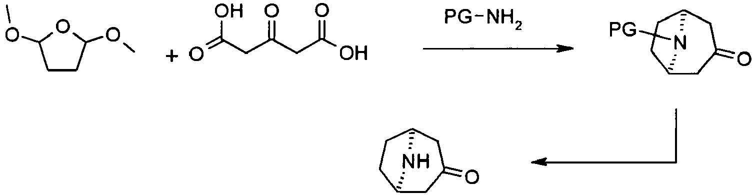 The protective group can be removed from the tropane ring by usual methods described in Protective groups in Organic Synthesis (Greene and Wuts, Third edition, Wiley Interscience, 1999) to prepare the free tropinone or the corresponding salt (generally HBr or HCl) depending on the deprotection methodology.

The preparation of compounds of general formula (I) may then be achieved by the sequence described in the next scheme from the previously described tropinone : 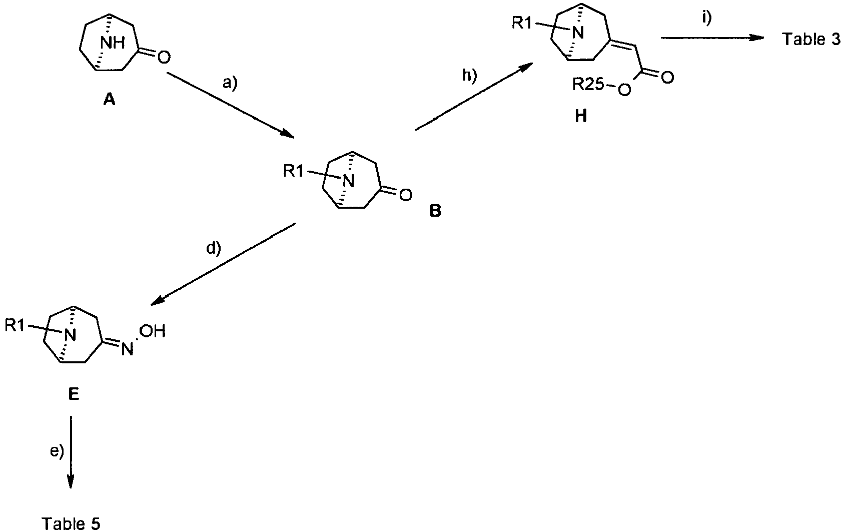
a) Tropinone A as free amine or salt can be reacted under basic conditions ; typically NaH, K₂CO₃, Na₂CO₃, Cs₂CO₃, NaOH, LiOH in a polar solvent such as DMF, DMAC, THF, NMP with an activated ring system R¹-X where R¹ was already described and X might be an halogen (Cl, I, Br, F) or a triflate at temperatures from ambient to boiling points of the corresponding solvents to generate the substituted tropane of formula B
   Alternatively, tropinone might be reacted with the same R¹-X under catalytic metal coupling reaction in the presence of a catalyst based on palladium species (e.g. Pd(TPP)₄), with a base such as an amine (e.g. triethylamine) or an alkali metal carbonate (e.g. K₂CO₃, Na₂CO₃, Cs₂CO₃) in a solvent such as toluene, DMF at from ambient temperature up to 140°C.
d) Compound of formula B can be transformed in the corresponding oximes of formula E by reacting with hydroxylamine hydrochloride in the presence of an alkali metal carbonate (e.g. K₂CO₃) or an organic base (e.g. triethylamine, pyridine) in an alcoholic solvent such as iPrOH or alternatively water at temperature from -20°C up to boiling point of solvent used.
e) Oximes of fomula E can be readily alkylated under basic conditions ; typically NaH, K₂CO₃, Na₂CO₃, Cs₂CO₃, NaOH, LiOH in a polar solvent such as DMF, DMAC, THF, NMP with an activated system R⁵-X where R⁵ is as already described and X might be an halogen (Cl, I, Br, F) or an other leaving group (e.g. Mesylate, N₂⁺) at temperatures from ambient to boiling points of the corresponding solvents to generate the substituted tropane of general formula described in table 5.
   The tropinone of type H might be prepared by a Knoevelagel type condensation of tropinone of type B and a malonate. In all these reaction, the two possible isomers could be further treated as mixture or isolated separately.
   The ester H can be reduced in the presence of a metal hydride (e.g. LiAlH₄) in an aprotic solvent (e.g. toluene, THF) to generate the allylic alcohol. These allylic alcohols might be activated and reacted to generate compounds listed in table 3.

It must be recognised that some reagents and reaction conditions may not be compatible with certain functionalities that may be present in the molecules described. In such cases it may be necessary to employ standard protection/deprotection protocols comprehensively reported in the literature and well known to a person skilled in the art.

In addition in some cases it may be necessary to perform further routine synthetic steps not described herein to complete the synthesis of the desired compounds. An artisan will also recognise that it may be possible to achieve the synthesis of the desired compounds by performing some of the steps of these synthetic routes in a different order to that described.

A person skilled in the art will also recognise that it may be possible to perform standard functional group interconversions or substitution reactions on the compounds described therein to introduce or modify substituents.

The compounds of formula (I) can be used to combat and control infestations of insect pests such as Lepidoptera, Diptera, Hemiptera, Thysanoptera, Orthoptera, Dictyoptera, Coleoptera, Siphonaptera, Hymenoptera and Isoptera and also other invertebrate pests, for example, acarine, nematode and mollusc pests. Insects, acarines, nematodes and molluscs are hereinafter collectively referred to as pests. The pests which may be combated and controlled by the use of the invention compounds include those pests associated with agriculture (which term includes the growing of crops for food and fibre products), horticulture and animal husbandry, companion animals, forestry and the storage of products of vegetable origin (such as fruit, grain and timber); those pests associated with the damage of man-made structures and the transmission of diseases of man and animals; and also nuisance pests (such as flies).

Examples of pest species which may be controlled by the compounds of formula (I) include: *Myzus persicae* (aphid), *Aphis gossypii* (aphid), *Aphis fabae* (aphid), *Lygus* spp. (capsids), *Dysdercus* spp. (capsids), *Nilaparvata lugens* (planthopper), *Nephotettixc incticeps* (leafhopper), *Nezara* spp. (stinkbugs), *Euschistus* spp. (stinkbugs), *Leptocorisa* spp. (stinkbugs), *Frankliniella occidentalis* (thrip), Thrips spp. (thrips), *Leptinotarsa decemlineata* (Colorado potato beetle), *Anthonomus grandis* (boll weevil), *Aonidiella* spp. (scale insects), *Trialeurodes* spp. (white flies), *Bemisia tabaci* (white fly), *Ostrinia nubilalis* (European corn borer), *Spodoptera littoralis* (cotton leafworm), *Heliothis virescens* (tobacco budworm), *Helicoverpa armigera* (cotton bollworm), *Helicoverpa zea* (cotton bollworm), *Sylepta derogata* (cotton leaf roller), *Pieris brassicae* (white butterfly), *Plutella xylostella* (diamond back moth), *Agrotis* spp. (cutworms), *Chilo suppressalis* (rice stem borer), *Locusta_migratoria* (locust), *Chortiocetes terminifera* (locust), *Diabrotica* spp. (rootworms), *Panonychus ulmi* (European red mite), *Panonychus citri* (citrus red mite), *Tetranychus urticae* (two-spotted spider mite), *Tetranychus cinnabarinus* (carmine spider mite), *Phyllocoptruta oleivora* (citrus rust mite),

*Polyphagotarsonemus latus* (broad mite), *Brevipalpus* spp. (flat mites), *Boophilus microplus* (cattle tick), *Dermacentor variabilis* (American dog tick), *Ctenocephalides felis* (cat flea), *Liriomyza* spp. (leafminer), *Musca domestica* (housefly), *Aedes aegypti* (mosquito), *Anopheles* spp. (mosquitoes), Culex spp. (mosquitoes), *Lucillia* spp. (blowflies), *Blattella germanica* (cockroach), *Periplaneta americana* (cockroach), *Blatta orientalis* (cockroach), termites of the Mastotermitidae (for example *Mastotermes* spp.), the Kalotermitidae (for example *Neotermes* spp.), the Rhinotermitidae (for example *Coptotermes formosanus, Reticulitermes flavipes, R. speratu, R. virginicus, R. hesperus,* and *R. santonensis*) and the Termitidae (for example *Globitermes sulphureus*), *Solenopsis geminata* (fire ant), *Monomorium pharaonis* (pharaoh's ant), *Damalinia* spp. and *Linognathus* spp. (biting and sucking lice), *Meloidogyne* spp. (root knot nematodes), *Globodera* spp. and *Heterodera* spp. (cyst nematodes), *Pratylenchus* spp. (lesion nematodes), *Rhodopholus* spp. (banana burrowing nematodes), *Tylenchulus* spp.(citrus nematodes), *Haemonchus contortus* (barber pole worm), *Caenorhabditis elegans*_(vinegar eelworm), *Trichostrongylus* spp. (gastro intestinal nematodes) and *Deroceras reticulatum* (slug).

The invention therefore provides a method of combating and controlling insects, acarines, nematodes or molluscs which comprises applying an insecticidally, acaricidally, nematicidally or molluscicidally effective amount of a compound of formula (I), or a composition containing a compound of formula (I), to a plant susceptible to attack by a pest, The compounds of formula (I) are preferably used against insects, acarines or nematodes.

The term "plant" as used herein includes seedlings, bushes and trees.

In order to apply a compound of formula (I) as an insecticide, acaricide, nematicide or molluscicide to a pest, a locus of pest, or to a plant susceptible to attack by a pest, a compound of formula (I) is usually formulated into a composition which includes, in addition to the compound of formula (I), a suitable inert diluent or carrier and, optionally, a surface active agent (SFA). SFAs are chemicals which are able to modify the properties of an interface (for example, liquid/solid, liquid/air or liquid/liquid interfaces) by lowering the interfacial tension and thereby leading to changes in other properties (for example dispersion, emulsification and wetting). It is preferred that all compositions (both solid and liquid formulations) comprise, by weight, 0.0001 to 95%, more preferably 1 to 85%, for example 5 to 60%, of a compound of formula (I). The composition is generally used for the control of pests such that a compound of formula (I) is applied at a rate of from 0.1g to 10kg per hectare, preferably from 1 g to 6kg per hectare, more preferably from 1 g to 1 kg per hectare.

When used in a seed dressing, a compound of formula (I) is used at a rate of 0.0001 g to 10g (for example 0.001 g or 0.05g), preferably 0.005g to 10g, more preferably 0.005g to 4g, per kilogram of seed.

In another aspect the present invention provides an insecticidal, acaricidal, nematicidal or molluscicidal composition comprising an insecticidally, acaricidally, nematicidally or molluscicidally effective amount of a compound of formula (I) and a suitable carrier or diluent therefor. The composition is preferably an insecticidal, acaricidal, nematicidal or molluscicidal composition.

The compounds of formula (I) are preferably used against insects, acarines or nematodes.

The compositions can be chosen from a number of formulation types, including dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, fogging/smoke formulations, capsule suspensions (CS) and seed treatment formulations. The formulation type chosen in any instance will depend upon the particular purpose envisaged and the physical, chemical and biological properties of the compound of formula (I).

Dustable powders (DP) may be prepared by mixing a compound of formula (I) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of formula (I) and one or more powdered solid diluents or carriers, or from pre-formed blank granules by absorbing a compound of formula (I) (or a solution thereof, in a suitable agent) in a porous granular material (such as pumice, attapulgite clays, fuller's earth, kieselguhr, diatomaceous earths or ground corn cobs) or by adsorbing a compound of formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of formula (I) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of formula (I) in an organic solvent (optionally containing one or more wetting agents, one or more emulsifying agents or a mixture of said agents). Suitable organic solvents for use in ECs include aromatic hydrocarbons (such as alkylbenzenes or alkyl naphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment. Preparation of an EW involves obtaining a compound of formula (I) either as a liquid (if it is not a liquid at room temperature, it may be melted at a reasonable temperature, typically below 70°C) or in solution (by dissolving it in an appropriate solvent) and then emulsifiying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SFAs, to produce spontaneously a thermodynamically stable isotropic liquid formulation. A compound of formula (I) is present initially in either the water or the solvent/SFA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of formula (I). SCs may be prepared by ball or bead milling the solid compound of formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of formula (I) may be dry milled and added to water, containing agents hereinbefore described, to produce the desired end product.

Aerosol formulations comprise a compound of formula (I) and a suitable propellant (for example *n*-butane). A compound of formula (I) may also be dissolved or dispersed in a suitable medium (for example water or a water miscible liquid, such as *n*-propanol) to provide compositions for use in non-pressurised, hand-actuated spray pumps.

A compound of formula (I) may be mixed in the dry state with a pyrotechnic mixture to form a composition suitable for generating, in an enclosed space, a smoke containing the compound.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of formula (I) and, optionally, a carrier or diluent therefor. The polymeric shell may be produced by either an interfacial polycondensation reaction or by a coacervation procedure. The compositions may provide for controlled release of the compound of formula (I) and they may be used for seed treatment. A compound of formula (I) may also be formulated in a biodegradable polymeric matrix to provide a slow, controlled release of the compound.

A composition may include one or more additives to improve the biological performance of the composition (for example by improving wetting, retention or distribution on surfaces; resistance to rain on treated surfaces; or uptake or mobility of a compound of formula (I)). Such additives include surface active agents, spray additives based on oils, for example certain mineral oils or natural plant oils (such as soy bean and rape seed oil), and blends of these with other bio-enhancing adjuvants (ingredients which may aid or modify the action of a compound of formula (I)).

A compound of formula (I) may also be formulated for use as a seed treatment, for example as a powder composition, including a powder for dry seed treatment (DS), a water soluble powder (SS) or a water dispersible powder for slurry treatment (WS), or as a liquid composition, including a flowable concentrate (FS), a solution (LS) or a capsule suspension (CS). The preparations of DS, SS, WS, FS and LS compositions are very similar to those of, respectively, DP, SP, WP, SC and DC compositions described above. Compositions for treating seed may include an agent for assisting the adhesion of the composition to the seed (for example a mineral oil or a film-forming barrier).

Wetting agents, dispersing agents and emulsifying agents may be surface SFAs of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di-*iso*propyl- and tri-isopropyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

A compound of formula (I) may be applied by any of the known means of applying pesticidal compounds. For example, it may be applied, formulated or unformulated, to the pests or to a locus of the pests (such as a habitat of the pests, or a growing plant liable to infestation by the pests) or to any part of the plant, including the foliage, stems, branches or roots, to the seed before it is planted or to other media in which plants are growing or are to be planted (such as soil surrounding the roots, the soil generally, paddy water or hydroponic culture systems), directly or it may be sprayed on, dusted on, applied by dipping, applied as a cream or paste formulation, applied as a vapour or applied through distribution or incorporation of a composition (such as a granular composition or a composition packed in a water-soluble bag) in soil or an aqueous environment.

A compound of formula (I) may also be injected into plants or sprayed onto vegetation using electrodynamic spraying techniques or other low volume methods, or applied by land or aerial irrigation systems.

Compositions for use as aqueous preparations (aqueous solutions or dispersions) are generally supplied in the form of a concentrate containing a high proportion of the active ingredient, the concentrate being added to water before use. These concentrates, which may include DCs, SCs, ECs, EWs, MEs SGs, SPs, WPs, WGs and CSs, are often required to withstand storage for prolonged periods and, after such storage, to be capable of addition to water to form aqueous preparations which remain homogeneous for a sufficient time to enable them to be applied by conventional spray equipment. Such aqueous preparations may contain varying amounts of a compound of formula (I) (for example 0.0001 to 10%, by weight) depending upon the purpose for which they are to be used.

A compound of formula (I) may be used in mixtures with fertilisers (for example nitrogen-, potassium- or phosphorus-containing fertilisers). Suitable formulation types include granules of fertiliser. The mixtures suitably contain up to 25% by weight of the compound of formula (I).

The invention therefore also provides a fertiliser composition comprising a fertiliser and a compound of formula (I).

The compositions of this invention may contain other compounds having biological activity, for example micronutrients or compounds having fungicidal activity or which possess plant growth regulating, herbicidal, insecticidal, nematicidal or acaricidal activity.

The compound of formula (I) may be the sole active ingredient of the composition or it may be admixed with one or more additional active ingredients such as a pesticide, fungicide, synergist, herbicide or plant growth regulator where appropriate. An additional active ingredient may: provide a composition having a broader spectrum of activity or increased persistence at a locus; synergise the activity or complement the activity (for example by increasing the speed of effect or overcoming repellency) of the compound of formula (I); or help to overcome or prevent the development of resistance to individual components. The particular additional active ingredient will depend upon the intended utility of the composition. Examples of suitable pesticides include the following:
a) Pyrethroids, such as permethrin, cypermethrin, fenvalerate, esfenvalerate, deltamethrin, cyhalothrin (in particular lambda-cyhalothrin), bifenthrin, fenpropathrin, cyfluthrin, tefluthrin, fish safe pyrethroids (for example ethofenprox), natural pyrethrin, tetramethrin, s-bioallethrin, fenfluthrin, prallethrin or 5-benzyl-3-furylmethyl-(E)-(1R,3S)-2,2-dimethyl- 3-(2-oxothiolan-3-ylidenemethyl)cycl opropane carboxylate;
b) Organophosphates, such as, profenofos, sulprofos, acephate, methyl parathion, azinphos-methyl, demeton-s-methyl, heptenophos, thiometon, fenamiphos, monocrotophos, profenofos, triazophos, methamidophos, dimethoate, phosphamidon, malathion, chlorpyrifos, phosalone, terbufos, fensulfothion, fonofos, phorate, phoxim, pirimiphos-methyl, pirimiphos-ethyl, fenitrothion, fosthiazate or diazinon;
c) Carbamates (including aryl carbamates), such as pirimicarb, triazamate, cloethocarb, carbofuran, furathiocarb, ethiofencarb, aldicarb, thiofurox, carbosulfan, bendiocarb, fenobucarb, propoxur, methomyl or oxamyl;
d) Benzoyl ureas, such as diflubenzuron, triflumuron, hexaflumuron, flufenoxuron or chlorfluazuron;
e) Organic tin compounds, such as cyhexatin, fenbutatin oxide or azocyclotin;
f) Pyrazoles, such as tebufenpyrad and fenpyroximate;
g) Macrolides, such as avermectins or milbemycins, for example abamectin, emamectin benzoate, ivermectin, milbemycin, spinosad or azadirachtin;
h) Hormones or pheromones;
i) Organochlorine compounds such as endosulfan, benzene hexachloride, DDT, chlordane or dieldrin;
j) Amidines, such as chlordimeform or amitraz;
k) Fumigant agents, such as chloropicrin, dichloropropane, methyl bromide or metam;
l) Chloronicotinyl compounds such as imidacloprid, thiacloprid, acetamiprid, nitenpyram or thiamethoxam;
m) Diacylhydrazines, such as tebufenozide, chromafenozide or methoxyfenozide;
n) Diphenyl ethers, such as diofenolan or pyriproxifen;
o) Indoxacarb;
p) Chlorfenapyr;
q) Pymetrozine;
r) Spirotetramat, Spiromesifen; or
s) Flubendiamid or Rynaxypyr

In addition to the major chemical classes of pesticide listed above, other pesticides having particular targets may be employed in the composition, if appropriate for the intended utility of the composition. For instance, selective insecticides for particular crops, for example stemborer specific insecticides (such as cartap) or hopper specific insecticides (such as buprofezin) for use in rice may be employed. Alternatively insecticides or acaricides specific for particular insect species/stages may also be included in the compositions (for example acaricidal ovo-larvicides, such as clofentezine, flubenzimine, hexythiazox or tetradifon; acaricidal motilicides, such as dicofol or propargite; acaricides, such as bromopropylate or chlorobenzilate; or growth regulators, such as hydramethylnon, cyromazine, methoprene, chlorfluazuron or diflubenzuron).

Examples of fungicidal compounds which may be included in the composition of the invention are (*E*)-*N*-methyl-2-[2-(2,5,dimethylphenoxymethyl)phenyl]-2-methoxy-iminoacetamide (SSF-129), 4-bromo-2-cyano-*N*,*N*-dimethyl-6-trifluoromethylbenzimidazole-1-sulphonamide, α-[*N*-(3-chloro-2,6-xylyl)-2-methoxy-acetamido]-γ-butyrolactone, 4-chloro-2-cyano-*N*,*N*-dimethyl-5-*p*-tolylimidazole-1-sulfonamide (IKF-916, cyamidazosulfamid), 3-5-dichloro-*N*-(3-chloro-1-ethyl-1-methyl-2-oxopropyl)-4-methylbenzamide (RH-7281, zoxamide), *N*-allyl-4,5,-dimethyl-2-trimethylsilylthiophene-3-carboxamide (MON65500), *N*-(1-cyano-1,2-dimethylpropyl)-2-(2,4-dichlorophenoxy)propionamide (AC382042), *N*-(2-methoxy-5-pyridyl)-cyclopropane carboxamide, acibenzolar (CGA245704), alanycarb, aldimorph, anilazine, azaconazole, azoxystrobin, benalaxyl, benomyl, biloxazol, bitertanol, blasticidin S, bromuconazole, bupirimate, captafol, captan, carbendazim, carbendazim chlorhydrate, carboxin, carpropamid, carvone, CGA41396, CGA41397, chinomethionate, chlorothalonil, chlorozolinate, clozylacon, copper containing compounds such as copper oxychloride, copper oxyquinolate, copper sulphate, copper tallate and Bordeaux mixture, cymoxanil, cyproconazole, cyprodinil, debacarb, di-2-pyridyl disulphide 1,1'-dioxide, dichlofluanid, diclomezine, dicloran, diethofencarb, difenoconazole, difenzoquat, diflumetorim, O,O-di-*iso*-propyl-*S*-benzyl thiophosphate, dimefluazole, dimetconazole, dimethomorph, dimethirimol, diniconazole, dinocap, dithianon, dodecyl dimethyl ammonium chloride, dodemorph, dodine, doguadine, edifenphos, epoxiconazole, ethirimol, ethyl(*Z*)-*N*-benzy)-*N*([methyl(methyl-thioethylideneamino-oxycarbonyl)amino]thio)-β-alaninate, etridiazole, famoxadone, fenamidone (RPA407213), fenarimol, fenbuconazole, fenfuram, fenhexamid (KBR2738), fenpiclonil, fenpropidin, fenpropimorph, fentin acetate, fentin hydroxide, ferbam, ferimzone, fluazinam, fludioxonil, flumetover, fluoroimide, fluquinconazole, flusilazole, flutolanil, flutriafol, folpet, fuberidazole, furalaxyl, furametpyr, guazatine, hexaconazole, hydroxyisoxazole, hymexazole, imazalil, imibenconazole, iminoctadine, iminoctadine triacetate, ipconazole, iprobenfos, iprodione, iprovalicarb (SZX0722), isopropanyl butyl carbamate, isoprothiolane, kasugamycin, kresoxim-methyl, LY186054, LY211795, LY248908, mancozeb, maneb, mefenoxam, mepanipyrim, mepronil, metalaxyl, metconazole, metiram, metiram-zinc, metominostrobin, myclobutanil, neoasozin, nickel dimethyldithiocarbamate, nitrothal-*iso*propyl, nuarimol, ofurace, organomercury compounds, oxadixyl, oxasulfuron, oxolinic acid, oxpoconazole, oxycarboxin, pefurazoate, penconazole, pencycuron, phenazin oxide, phosetyl-Al, phosphorus acids, phthalide, picoxystrobin (ZA1963), polyoxin D, polyram, probenazole, prochloraz, procymidone, propamocarb, propiconazole, propineb, propionic acid, pyrazophos, pyrifenox, pyrimethanil, pyroquilon, pyroxyfur, pyrrolnitrin, quaternary ammonium compounds, quinomethionate, quinoxyfen, quintozene, sipconazole (F-155), sodium pentachlorophenate, spiroxamine, streptomycin, sulphur, tebuconazole, tecloftalam, tecnazene, tetraconazole, thiabendazole, thifluzamid, 2-(thiocyanomethylthio)benzothiazole, thiophanate-methyl, thiram, timibenconazole, tolclofos-methyl, tolylfluanid, triadimefon, triadimenol, triazbutil, triazoxide, tricyclazole, tridemorph, trifloxystrobin (CGA279202), triforine, triflumizole, triticonazole, validamycin A, vapam, vinclozolin, zineb and ziram.

The compounds of formula (I) may be mixed with soil, peat or other rooting media for the protection of plants against seed-borne, soil-borne or foliar fungal diseases.

Examples of suitable synergists for use in the compositions include piperonyl butoxide, sesamex, safroxan and dodecyl imidazole.

Suitable herbicides and plant-growth regulators for inclusion in the compositions will depend upon the intended target and the effect required.

An example of a rice selective herbicide which may be included is propanil. An example of a plant growth regulator for use in cotton is PIX™.

Some mixtures may comprise active ingredients which have significantly different physical, chemical or biological properties such that they do not easily lend themselves to the same conventional formulation type. In these circumstances other formulation types may be prepared. For example, where one active ingredient is a water insoluble solid and the other a water insoluble liquid, it may nevertheless be possible to disperse each active ingredient in the same continuous aqueous phase by dispersing the solid active ingredient as a suspension (using a preparation analogous to that of an SC) but dispersing the liquid active ingredient as an emulsion (using a preparation analogous to that of an EW). The resultant composition is a suspoemulsion (SE) formulation.

The compounds of formula (1) are also active fungicides and may be used to control one or more of the following pathogens: *Pyricularia oryzae* (*Magnaporthe grisea*) on rice and wheat and other *Pyricularia* spp. on other hosts; *Puccinia triticina* (or *recondita*), *Puccinia striiformis* and other rusts on wheat, *Puccinia hordei, Puccinia striiformis* and other rusts on barley, and rusts on other hosts (for example turf, rye, coffee, pears, apples, peanuts, sugar beet, vegetables and ornamental plants); *Erysiphe cichoracearum* on cucurbits (for example melon); *Blumeria* (or *Erysiphe*) *graminis* (powdery mildew) on barley, wheat, rye and turf and other powdery mildews on various hosts, such as *Sphaerotheca macularis* on hops, *Sphaerotheca fusca (Sphaerotheca fuliginea*) on cucurbits (for example cucumber), *Leveillula taurica* on tomatoes, aubergine and green pepper, *Podosphaera leucotricha* on apples and *Uncinula necator* on vines; *Cochliobolus* spp., *Helminthosporium* spp., *Drechslera* spp. (*Pyrenophora* spp.), *Rhynchosporium* spp., *Mycosphaerella graminicola* (*Septoria tritici*) and *Phaeosphaeria nodorum* (*Stagonospora nodorum* or *Septoria nodorum*), *Pseudocercosporella herpotrichoides* and *Gaeumannomyces graminis* on cereals (for example wheat, barley, rye), turf and other hosts; *Cercospora arachidicola* and *Cercosporidium personatum* on peanuts and other *Cercospora* spp. on other hosts, for example sugar beet, bananas, soya beans and rice; *Botrytis cinerea* (grey mould) on tomatoes, strawberries, vegetables, vines and other hosts and other *Botrytis* spp. on other hosts; *Alternaria* spp. on vegetables (for example carrots), oil-seed rape, apples, tomatoes, potatoes, cereals (for example wheat) and other hosts; *Venturia* spp. (including *Venturia inaequalis* (scab)) on apples, pears, stone fruit, tree nuts and other hosts; *Cladosporium* spp. on a range of hosts including cereals (for example wheat) and tomatoes; *Monilinia* spp. on stone fruit, tree nuts and other hosts; *Didymella* spp. on tomatoes, turf, wheat, cucurbits and other hosts; *Phoma* spp. on oil-seed rape, turf, rice, potatoes, wheat and other hosts; *Aspergillus* spp. and *Aureobasidium* spp. on wheat, lumber and other hosts; *Ascochyta* spp. on peas, wheat, barley and other hosts; *Stemphylium* spp. (*Pleospora* spp.) on apples, pears, onions and other hosts; summer diseases (for example bitter rot (*Glomerella cingulata*), black rot or frogeye leaf spot (*Botryosphaeria obtusa*), Brooks fruit spot *(Mycosphaerella pomi*), Cedar apple rust (*Gymnosporangium juniperi-virginianae*), sooty blotch (*Gloeodes pomigena*), flyspeck (*Schizothyrium pomi*) and white rot (*Botryosphaeria dothidea*)) on apples and pears; *Plasmopara viticola* on vines; other downy mildews, such as *Bremia lactucae* on lettuce, *Peronospora* spp. on soybeans, tobacco, onions and other hosts, *Pseudoperonospora humuli* on hops and *Pseudoperonospora cubensis* on cucurbits; *Pythium* spp. (including *Pythium ultimum)* on turf and other hosts; *Phytophthora infestans* on potatoes and tomatoes and other *Phytophthora* spp. on vegetables, strawberries, avocado, pepper, ornamentals, tobacco, cocoa and other hosts; *Thanatephorus cucumeris* on rice and turf and other *Rhizoctonia* spp. on various hosts such as wheat and barley, peanuts, vegetables, cotton and turf; *Sclerotinia* spp. on turf, peanuts, potatoes, oil-seed rape and other hosts; *Sclerotium* spp. on turf, peanuts and other hosts; *Gibberella fujikuroi* on rice; *Colletotrichum* spp. on a range of hosts including turf, coffee and vegetables; *Laetisaria fuciformis* on turf; *Mycosphaerella* spp. on bananas, peanuts, citrus, pecans, papaya and other hosts; *Diaporthe* spp. on citrus, soybean, melon, pears, lupin and other hosts; *Elsinoe* spp. on citrus, vines, olives, pecans, roses and other hosts; *Verticillium* spp. on a range of hosts including hops, potatoes and tomatoes; *Pyrenopeziza* spp. on oil-seed rape and other hosts; *Oncobasidium theobromae* on cocoa causing vascular streak dieback; *Fusarium* spp., *Typhula* spp., *Microdochium nivale, Ustilago* spp., *Urocystis* spp., *Tilletia* spp. and *Claviceps purpurea* on a variety of hosts but particularly wheat, barley, turf and maize; *Ramularia* spp. on sugar beet, barley and other hosts; post-harvest diseases particularly of fruit (for example *Penicillium digitatum, Penicillium italicum* and *Trichoderma viride* on oranges, *Colletotrichum musae* and *Gloeosporium musarum* on bananas and *Botrytis cinerea* on grapes); other pathogens on vines, notably *Eutypa lata, Guignardia bidwellii, Phellinus igniarus, Phomopsis viticola, Pseudopeziza tracheiphila* and *Stereum hirsutum;* other pathogens on trees (for example *Lophodermium seditiosum*) or lumber, notably *Cephaloascus fragrans, Ceratocystis* spp., *Ophiostoma piceae, Penicillium* spp., *Trichoderma pseudokoningii, Trichoderma viride, Trichoderma harzianum, Aspergillus niger, Leptographium lindbergi* and *Aureobasidium pullulans*; and fungal vectors of viral diseases (for example *Polymyxa graminis* on cereals as the vector of barley yellow mosaic virus (BYMV) and *Polymyxa betae* on sugar beet as the vector of rhizomania).

The compounds of formula (1) show particularly good activity against the Oomycete class of pathogens such as *Phytophthora infestans, Plasmopara* species, e.g. *Plasmopara viticola* and *Pythium* species e.g. *Pythium ultimum.*

A compound of formula (1) may move acropetally, basipetally or locally in plant tissue to be active against one or more fungi. Moreover, a compound of formula (1) may be volatile enough to be active in the vapour phase against one or more fungi on the plant.

The invention therefore provides a method of combating or controlling phytopathogenic fungi which comprises applying a fungicidally effective amount of a compound of formula (1), or a composition containing a compound of formula (1), to a plant, to a seed of a plant, to the locus of the plant or seed or to soil or any other plant growth medium, e.g. nutrient solution.

The term "plant" as used herein includes seedlings, bushes and trees. Furthermore, the fungicidal method of the invention includes protectant, curative, systemic, eradicant and antisporulant treatments.

The compounds of formula (1) are preferably used for agricultural, horticultural and turfgrass purposes in the form of a composition.

In order to apply a compound of formula (1) to a plant, to a seed of a plant, to the locus of the plant or seed or to soil or any other growth medium, a compound of formula (1) is usually formulated into a composition which includes, in addition to the compound of formula (1), a suitable inert diluent or carrier and, optionally, a surface active agent (SFA). SFAs are chemicals that are able to modify the properties of an interface (for example, liquid/solid, liquid/air or liquid/liquid interfaces) by lowering the interfacial tension and thereby leading to changes in other properties (for example dispersion, emulsification and wetting). It is preferred that all compositions (both solid and liquid formulations) comprise, by weight, 0.0001 to 95%, more preferably 1 to 85%, for example 5 to 60%, of a compound of formula (1). The composition is generally used for the control of fungi such that a compound of formula (1) is applied at a rate of from 0.1 g to 10kg per hectare, preferably from 1 g to 6kg per hectare, more preferably from 1 g to 1 kg per hectare.

When used in a seed dressing, a compound of formula (1) is used at a rate of 0.0001g to 10g (for example 0.001g or 0.05g), preferably 0.005g to 10g, more preferably 0.005g to 4g, per kilogram of seed.

In another aspect the present invention provides a fungicidal composition comprising a fungicidally effective amount of a compound of formula (1) and a suitable carrier or diluent therefor.

In a still further aspect the invention provides a method of combating and controlling fungi at a locus, which comprises treating the fungi, or the locus of the fungi with a fungicidally effective amount of a composition comprising a compound of formula (1).

The compositions can be chosen from a number of formulation types, including dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, fogging/smoke formulations, capsule suspensions (CS) and seed treatment formulations. The formulation type chosen in any instance will depend upon the particular purpose envisaged and the physical, chemical and biological properties of the compound of formula (1).

Dustable powders (DP) may be prepared by mixing a compound of formula (1) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of formula (1) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of formula (1) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of formula (1) and one or more powdered solid diluents or carriers, or from pre-formed blank granules by absorbing a compound of formula (1) (or a solution thereof, in a suitable agent) in a porous granular material (such as pumice, attapulgite clays, fuller's earth, kieselguhr, diatomaceous earths or ground corn cobs) or by adsorbing a compound of formula (1) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of formula (1) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of formula (1) in an organic solvent (optionally containing one or more wetting agents, one or more emulsifying agents or a mixture of said agents). Suitable organic solvents for use in ECs include aromatic hydrocarbons (such as alkylbenzenes or alkyl naphthalenes, exemplified by SOLVESSO 100, SOLVESSO 150 and SOLVESSO 200; SOLVESSO is a Registered Trade Mark), ketones (such as cyclohexanone or methylcyclohexanone), alcohols (such as benzyl alcohol, furfuryl alcohol or butanol), N-alkylpyrrolidones (such as *N*-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment. Preparation of an EW involves obtaining a compound of formula (1) either as a liquid (if it is not a liquid at ambient temperature, it may be melted at a reasonable temperature, typically below 70°C) or in solution (by dissolving it in an appropriate solvent) and then emulsifying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents that have a low solubility in water.

Microemulsions (ME) may be prepared by mixing water with a blend of one or more solvents with one or more SFAs, to produce spontaneously a thermodynamically stable isotropic liquid formulation. A compound of formula (1) is present initially in either the water or the solvent/SFA blend. Suitable solvents for use in MEs include those hereinbefore described for use in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of formula (1). SCs may be prepared by ball or bead milling the solid compound of formula (1) in a suitable medium, optionally with one or more dispersing agents, to produce a fine particle suspension of the compound. One or more wetting agents may be included in the composition and a suspending agent may be included to reduce the rate at which the particles settle. Alternatively, a compound of formula (1) may be dry milled and added to water, containing agents hereinbefore described, to produce the desired end product.

Aerosol formulations comprise a compound of formula (1) and a suitable propellant (for example *n*-butane). A compound of formula (1) may also be dissolved or dispersed in a suitable medium (for example water or a water miscible liquid, such as *n*-propanol) to provide compositions for use in non-pressurised, hand-actuated spray pumps.

A compound of formula (1) may be mixed in the dry state with a pyrotechnic mixture to form a composition suitable for generating, in an enclosed space, a smoke containing the compound.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of formula (1) and, optionally, a carrier or diluent therefor. The polymeric shell may be produced by either an interfacial polycondensation reaction or by a coacervation procedure. The compositions may provide for controlled release of the compound of formula (1) and they may be used for seed treatment. A compound of formula (1) may also be formulated in a biodegradable polymeric matrix to provide a slow, controlled release of the compound.

A composition may include one or more additives to improve the biological performance of the composition (for example by improving wetting, retention or distribution on surfaces; resistance to rain on treated surfaces; or uptake or mobility of a compound of formula (1)). Such additives include surface active agents, spray additives based on oils, for example certain mineral oils or natural plant oils (such as soy bean and rape seed oil), and blends of these with other bio-enhancing adjuvants (ingredients which may aid or modify the action of a compound of formula (1)).

A compound of formula (1) may also be formulated for use as a seed treatment, for example as a powder composition, including a powder for dry seed treatment (DS), a water soluble powder (SS) or a water dispersible powder for slurry treatment (WS), or as a liquid composition, including a flowable concentrate (FS), a solution (LS) or a capsule suspension (CS). The preparations of DS, SS, WS, FS and LS compositions are very similar to those of, respectively, DP, SP, WP, SC and DC compositions described above. Compositions for treating seed may include an agent for assisting the adhesion of the composition to the seed (for example a mineral oil or a film-forming barrier).

Wetting agents, dispersing agents and emulsifying agents may be SFAs of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di-*iso*propyl- and tri-isopropyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefin sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

A compound of formula (1) may be applied by any of the known means of applying fungicidal compounds. For example, it may be applied, formulated or unformulated, to any part of the plant, including the foliage, stems, branches or roots, to the seed before it is planted or to other media in which plants are growing or are to be planted (such as soil surrounding the roots, the soil generally, paddy water or hydroponic culture systems), directly or it may be sprayed on, dusted on, applied by dipping, applied as a cream or paste formulation, applied as a vapour or applied through distribution or incorporation of a composition (such as a granular composition or a composition packed in a water-soluble bag) in soil or an aqueous environment.

A compound of formula (1) may also be injected into plants or sprayed onto vegetation using electrodynamic spraying techniques or other low volume methods, or applied by land or aerial irrigation systems.

Compositions for use as aqueous preparations (aqueous solutions or dispersions) are generally supplied in the form of a concentrate containing a high proportion of the active ingredient, the concentrate being added to water before use. These concentrates, which may include DCs, SCs, ECs, EWs, MEs, SGs, SPs, WPs, WGs and CSs, are often required to withstand storage for prolonged periods and, after such storage, to be capable of addition to water to form aqueous preparations which remain homogeneous for a sufficient time to enable them to be applied by conventional spray equipment. Such aqueous preparations may contain varying amounts of a compound of formula (1) (for example 0.0001 to 10%, by weight) depending upon the purpose for which they are to be used.

A compound of formula (1) may be used in mixtures with fertilisers (for example nitrogen-, potassium- or phosphorus-containing fertilisers). Suitable formulation types include granules of fertiliser. The mixtures suitably contain up to 25% by weight of the compound of formula (1).

The invention therefore also provides a fertiliser composition comprising a fertiliser and a compound of formula (1).

The compositions of this invention may contain other compounds having biological activity, for example micronutrients or compounds having similar or complementary fungicidal activity or which possess plant growth regulating, herbicidal, insecticidal, nematicidal or acaricidal activity.

By including another fungicide, the resulting composition may have a broader spectrum of activity or a greater level of intrinsic activity than the compound of formula (1) alone. Further the other fungicide may have a synergistic effect on the fungicidal activity of the compound of formula (1).

The compound of formula (1) may be the sole active ingredient of the composition or it may be admixed with one or more additional active ingredients such as a pesticide, fungicide, synergist, herbicide or plant growth regulator where appropriate. An additional active ingredient may: provide a composition having a broader spectrum of activity or increased persistence at a locus; synergise the activity or complement the activity (for example by increasing the speed of effect or overcoming repellency) of the compound of formula (1); or help to overcome or prevent the development of resistance to individual components. The particular additional active ingredient will depend upon the intended utility of the composition.

Examples of fungicidal compounds which may be included in the composition of the invention are AC 382042 (*N*-(1-cyano-1,2-dimethylpropyl)-2-(2,4-dichloro-phenoxy) propionamide), acibenzolar-S-methyl, alanycarb, aldimorph, anilazine, azaconazole, azafenidin, azoxystrobin, benalaxyl, benomyl, benthiavalicarb, biloxazol, bitertanol, blasticidin S, boscalid (new name for nicobifen), bromuconazole, bupirimate, captafol, captan, carbendazim, carbendazim chlorhydrate, carboxin, carpropamid, carvone, CGA 41396, CGA 41397, chinomethionate, chlorbenzthiazone, chlorothalonil, chlorozolinate, clozylacon, copper containing compounds such as copper oxychloride, copper oxyquinolate, copper sulphate, copper tallate, and Bordeaux mixture, cyamidazosulfamid, cyazofamid (IKF-916), cyflufenamid, cymoxanil, cyproconazole, cyprodinil, debacarb, di-2-pyridyl disulphide 1,1'-dioxide, dichlofluanid, diclocymet, diclomezine, dicloran, diethofencarb, difenoconazole, difenzoquat, diflumetorim, *O*,*O*-di-*iso*-propyl-*S*-benzyl thiophosphate, dimefluazole, dimetconazole, dimethirimol, dimethomorph, dimoxystrobin, diniconazole, dinocap, dithianon, dodecyl dimethyl ammonium chloride, dodemorph, dodine, doguadine, edifenphos, epoxiconazole, ethaboxam, ethirimol, ethyl (*Z*)-*N*-benzyl-*N*([methyl(methyl-thioethylideneaminooxycarbonyl)amino]thio)-β-alaninate, etridiazole, famoxadone, fenamidone, fenarimol, fenbuconazole, fenfuram, fenhexamid, fenoxanil (AC 382042), fenpiclonil, fenpropidin, fenpropimorph, fentin acetate, fentin hydroxide, ferbam, ferimzone, fluazinam, fludioxonil, flumetover, flumorph, fluoroimide, fluoxastrobin, fluquinconazole, flusilazole, flusulfamide, flutolanil, flutriafol, folpet, fosetyl-aluminium, fuberidazole, furalaxyl, furametpyr, guazatine, hexaconazole, hydroxyisoxazole, hymexazole, imazalil, imibenconazole, iminoctadine, iminoctadine triacetate, ipconazole, iprobenfos, iprodione, iprovalicarb, isopropanyl butyl carbamate, isoprothiolane, kasugamycin, kresoxim-methyl, LY186054, LY211795, LY 248908, mancozeb, maneb, mefenoxam, mepanipyrim, mepronil, metalaxyl, metalaxyl M, metconazole, metiram, metiram-zinc, metominostrobin, metrafenone, MON65500 (*N*-allyl-4,5-dimethyl-2-trimethylsilylthiophene-3-carboxamide), myclobutanil, NTN0301, neoasozin, nickel dimethyldithiocarbamate, nitrothale-isopropyl, nuarimol, ofurace, organomercury compounds, orysastrobin, oxadixyl, oxasulfuron, oxolinic acid, oxpoconazole, oxycarboxin, pefurazoate, penconazole, pencycuron, phenazin oxide, phosphorus acids, phthalide, picoxystrobin, polyoxin D, polyram, probenazole, prochloraz, procymidone, propamocarb, propamocarb hydrochloride, propiconazole, propineb, propionic acid, proquinazid, prothioconazole, pyraclostrobin, pyrazophos, pyrifenox, pyrimethanil, pyroquilon, pyroxyfur, pyrrolnitrin, quaternary ammonium compounds, quinomethionate, quinoxyfen, quintozene, silthiofam (MON 65500), S-imazalil, simeconazole, sipconazole, sodium pentachlorophenate, spiroxamine, streptomycin, sulphur, tebuconazole, tecloftalam, tecnazene, tetraconazole, thiabendazole, thifluzamide, 2-(thiocyanomethylthio)benzothiazole, thiophanate-methyl, thiram, tiadinil, timibenconazole, tolclofos-methyl, tolylfluanid, triadimefon, triadimenol, triazbutil, triazoxide, tricyclazole, tridemorph, trifloxystrobin, triflumizole, triforine, triticonazole, validamycin A, vapam, vinclozolin, XRD-563, zineb, ziram, zoxamide and compounds of the formulae:

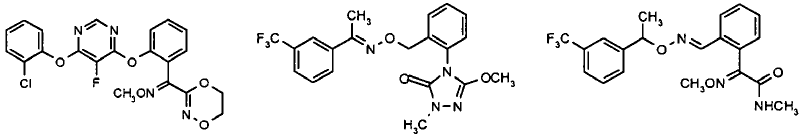

The compounds of formula (I) may be mixed with soil, peat or other rooting media for the protection of plants against seed-borne, soil-borne or foliar fungal diseases.

Some mixtures may comprise active ingredients, which have significantly different physical, chemical or biological properties such that they do not easily lend themselves to the same conventional formulation type. In these circumstances other formulation types may be prepared. For example, where one active ingredient is a water insoluble solid and the other a water insoluble liquid, it may nevertheless be possible to disperse each active ingredient in the same continuous aqueous phase by dispersing the solid active ingredient as a suspension (using a preparation analogous to that of an SC) but dispersing the liquid active ingredient as an emulsion (using a preparation analogous to that of an EW). The resultant composition is a suspoemulsion (SE) formulation.

Further areas of use of the compositions according to the invention are the protection of stored goods and storerooms and the protection of raw materials, such as wood, textiles, floor coverings or buildings, and also in the hygiene sector.

The invention also relates to the Use of a compound of formula (I) in the manufacture of a medicament for controlling pests on domestic animals and productive livestock.

In the hygiene sector, the compositions according to the invention are active against ectoparasites such as hard ticks, soft ticks, mange mites, harvest mites, flies (biting and licking), parasitic fly larvae, lice, hair lice, bird lice and fleas.

Examples of such parasites are:

Of the order Anoplurida: Haematopinus spp., Linognathus spp., Pediculus spp. and Phtirus spp., Solenopotes spp..

Of the order Mallophagida: Trimenopon spp., Menopon spp., Trinoton spp., Bovicola spp., Werneckiella spp., Lepikentron spp., Damalina spp., Trichodectes spp. and Felicola spp..

Of the order Diptera and the suborders Nematocerina and Brachycerina, for example Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp. and Melophagus spp..

Of the order Siphonapterida, for example Pulex spp., Ctenocephalides spp., Xenopsylla spp., Ceratophyllus spp..

Of the order Heteropterida, for example Cimex spp., Triatoma spp., Rhodnius spp., Panstrongylus spp..

Of the order Blattarida, for example Blatta orientalis, Periplaneta americana, Blattelagermanica and Supella spp..

Of the subclass Acaria (Acarida) and the orders Meta- and Meso-stigmata, for example Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp. and Varroa spp..

Of the orders Actinedida (Prostigmata) and Acaridida (Astigmata), for example Acarapis spp., Cheyletiella spp., Ornithocheyletia spp., Myobia spp., Psorergatesspp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp. and Laminosioptes spp..

The invention is illustrated by the following Examples:

### EXAMPLE 1

### 8-(5-Chloro-6-ethyl-pyrimidin-4-yl)-8-aza-bicyclo[3.2.1]octan-3-one O-(4-bromo-2-fluoro-benzyl)-oxime F

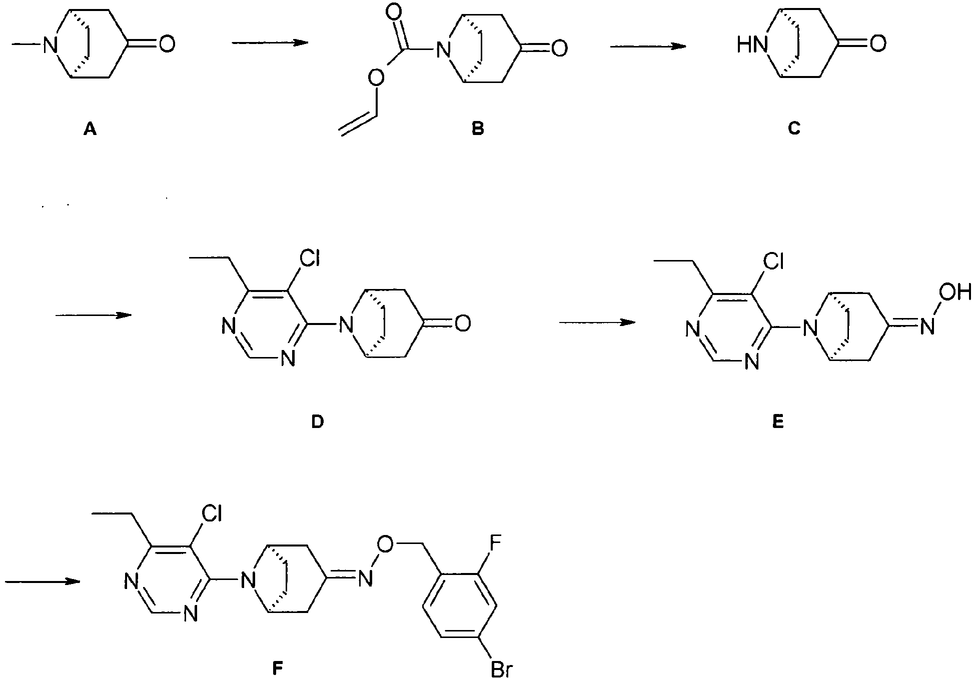

### Preparation of 3-Oxo-8-aza-bicyclo[3.2.1]octane-8-carboxylic acid vinyl ester B

To a solution of 37.6 g tropinone in 180 ml THF 57.5 g of vinyl chloroformate were added at 0°. The resulting suspension was stirred for 1 hour at ambient temperature and for 1 hour at reflux. At room temperature 400 ml of a saturated aqueous solution of NaHCO₃ were slowly added. The resulting mixture was stirred for 15 minutes and then extracted with ethyl acetate. The combined organic phases were dried over sodium sulphate and evaporated. The resulting 57.7 g of an orange oil which crystallized overnight was purified by column chromatography on silica gel (hexane-ethyl acetate 3:1 → 2:1) afforded the product 3-Oxo-8-aza-bicyclo[3.2.1]octane-8-carboxylic acid vinyl ester as a white solid (52.7 g).

¹H NMR (300 MHz, CDCl₃) 1.70 (m, 2H), 2.15 (m, 2H), 2.40 (m, 2H), 2.70 (m, 2H), 4.55 (d, 1H), 4.65 (s, 2H), 4.75 (d, 1H), 7.25 (dd, 1H).

### Preparation of 8-Aza-bicyclo[3.2.1]octan-3-one C

100 ml HCl 37% were added drop-wise to a solution of 48 g of crude 3-Oxo-8-aza-bicyclo[3.2.1]octane-8-carboxylic acid vinyl ester in 200 ml methanol. The resulting reaction mixture was stirred for one hour at reflux. The methanol was distilled off and the aqueous phase was neutralized with saturated aqueous solution of sodium carbonate. The resulting mixture was extracted with dichloromethane. The combined organic phases were dried over sodium sulphate and evaporated. The resulting orange crystalline product (8.9 g) was used in the next step without further purification.

¹H NMR (300 MHz, CDCl₃) 1.70 (m, 2H), 1.90 (m, 3H), 2.32 (m, 2H), 2.58 (m, 2H), 3.85 (s, 2H).

### Preparation of 8-(5-Chloro-6-ethyl-pyrimidin-4-yl)-8-aza-bicyclo[3.2.1]octan-3-one D

2.6 g potassium carbonate was added to a solution of 2.0 g 8-Aza-bicyclo[3.2.1]octan-3-one and 2.8 g 4,5-dichloro-6-ethylpyrimidine in 20 ml DMF at ambient temperature. The resulting mixture was stirred for 3 hours at 65°. The reaction mixture was poured on ice/water and extracted with ethyl acetate. The combined organic phases were dried over sodium sulphate and evaporated. Purification of the residue by column chromatography on silica gel (hexane-ethyl acetate 3:1) afforded the product 8-(5-Chloro-6-ethyl-pyrimidin-4-yl)-8-aza-bicyclo[3.2.1]octan-3-one as yellow oil (2.8 g).

¹H NMR (300 MHz, CDCl₃) 1.30 (t, 3H), 1.82 (m, 2H), 2.18 (m, 2H), 2.42 (m, 2H), 2.88 (m, 4H), 5.25 (s, 2H), 5.20 (s, 1H), 8.50 (s, 1H).

### Preparation of 8-(5-Chloro-6-ethyl-pyrimidin-4-yl)-8-aza-bicyclo[3.2.1]octan-3-one oxime E

236 mg hydroxylamine hydrochloride was added to a solution of 900 mg 8-(5-Chloro-6-ethyl-pyrimidin-4-yl)-8-aza-bicyclo[3.2.1]octan-3-one in 20 ml methanol/water 1:1. 556 mg of sodium acetate were added and the suspension was stirred for 2.5 hours at ambient temperature. The reaction mixture was poured on water and extracted with ethyl acetate. The combined organic phases were dried over sodium sulphate and evaporated. The resulting white crystalline product (950 mg) was used in the next step without further purification.

¹H NMR (300 MHz, CDCl₃) 1.30 (t, 3H), 1.78 (m, 2H), 2.10 (m, 2H), 2.40 (m, 2H), 2.73 (d, 1H), 2.90 (q, 2H), 3.25 (d, 1H) 5.13 (s, 1H), 5.20 (s, 1H), 8.50 (s, 1H).

### Preparation of 8-(5-Chloro-6-ethyl-pyrimidin-4-yl)-8-aza-bicyclo[3.2.1]octan-3-one O-(4-bromo-2-fluoro-benzyl)-oxime F

162 mg sodium hydride (50%) was added in portions to a solution of 860 mg 8-(5-Chloro-6-ethyl-pyrimidin-4-yl)-8-aza-bicyclo[3.2.1]octan-3-one oxime in 15 ml DMF at 0°. The resulting solution was stirred for 20 minutes at 0-5°, then 904 mg 4-bromo-2-fluorobenzylbromide was added. The reaction mixture was stirred for 16 hours at ambient temperature. 15 ml of a saturated aqueous ammonium chloride solution were added at 0-5°. The reaction mixture was poured on water and extracted with ethyl acetate. The combined organic phases were dried over sodium sulphate and evaporated. The resulting product was purified by preparative HPLC to yield 807 mg 8-(5-Chloro-6-ethyl-pyrimidin-4-yl)-8-aza-bicyclo[3.2.1]octan-3-one-O-(4-bromo-2-fluoro-benzyl)-oxime as yellow oil.

¹H NMR (300 MHz, CDCl₃) 1.28 (t, 3H), 1.70 (m, 2H), 2.05 (m, 2H), 2.35 (m, 2H), 2.78 (d, 1H), 2.85 (q, 2H), 3.15 (d, 1H) 5.05 (m, 3H), 5.15 (s, 1H), 7.22 (m, 3H), 8.45 (s, 1H).

The following compounds were prepared according to procedures analogous to those described in Example 1:

**Table 3**

| 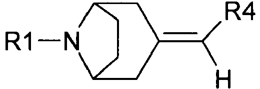 | | | |
|---|---|---|---|
| **Nr.** | **R1** | **R4** | **mp (°C)** |
| **3.1** | 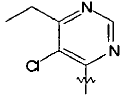 | 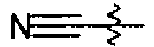 | **73** |
| **3.2** | 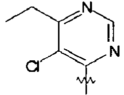 | 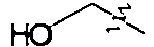 | |
| **3.3** | 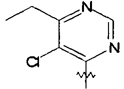 | 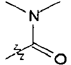 | |
| **3.4** | 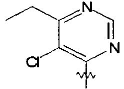 | 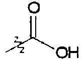 | **162** |
| **3.5** | 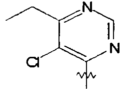 | 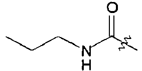 | |
| **3.6** | 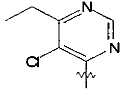 | 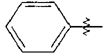 | |
| **3.7** | 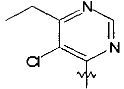 | 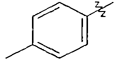 | |
| **3.8** | 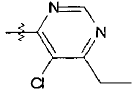 | 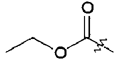 | |
| **3.9** | 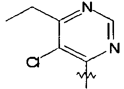 | 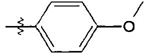 | |
| **3.10** | 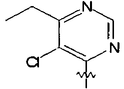 | 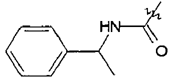 | |
| **3.11** | 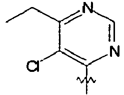 | 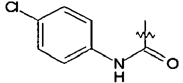 | |
| **3.12** | 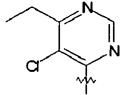 | 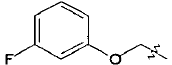 | |
| **3.13** | 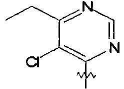 | 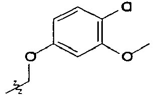 | |
| **3.14** | 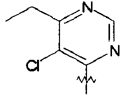 | 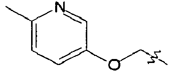 | |
| **3.15** | 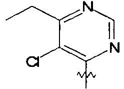 | 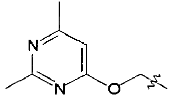 | |
| **3.16** | 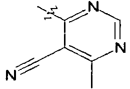 | 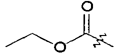 | |
| **3.17** | 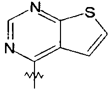 | 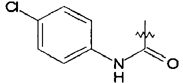 | |
| **3.18** | 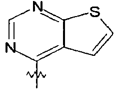 | 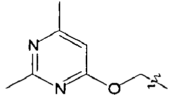 | |
| **3.19** | 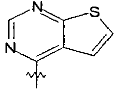 | 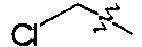 | |
| **3.20** | 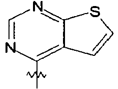 | 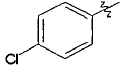 | |
| **3.21** | 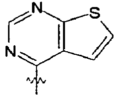 | 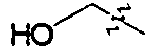 | |
| **3.22** | 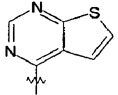 | 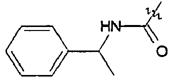 | |
| **3.23** | 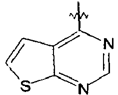 | 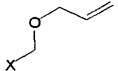 | |
| **3.24** | 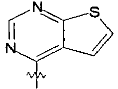 | 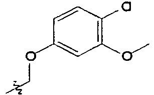 | |
| **3.26** | 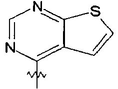 | 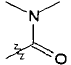 | |
| **3.26** | 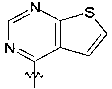 | 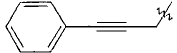 | |
| **3.27** | 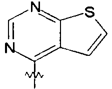 | 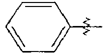 | |
| **3.28** | 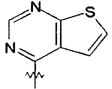 | 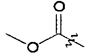 | |
| **3.29** | 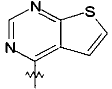 | 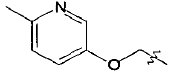 | |
| **3.30** | 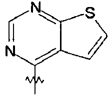 | 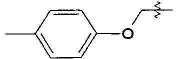 | |
| **3.31** | 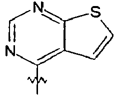 | 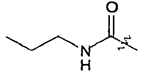 | |
| **3.32** | 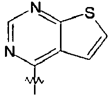 | 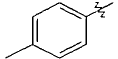 | |
| **3.33** | 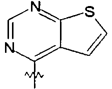 | 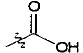 | |
| **3.34** | 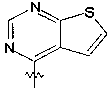 | 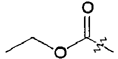 | **101** |
| **3.36** | 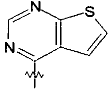 | 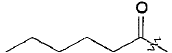 | |
| **6.36** | 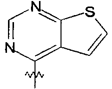 | 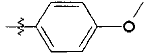 | |
| **3.37** | 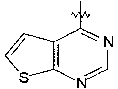 | 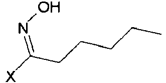 | **127** |
| **3.38** | 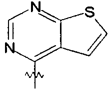 | 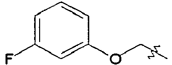 | |
| **3.39** | 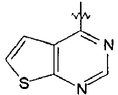 | 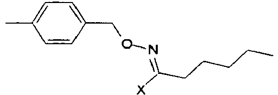 | |
| **3.40** | 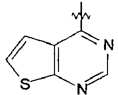 | 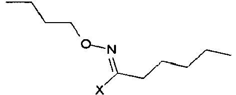 | |
| **3.41** | 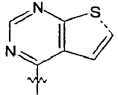 | 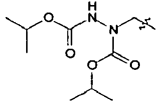 | |

**Table 5**

| 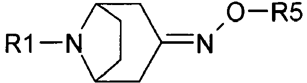 | | | |
|---|---|---|---|
| **5.1** | 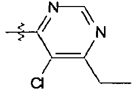 | 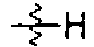 | **73** |
| **5.2** | 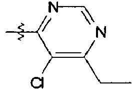 | 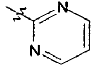 | |
| **5.3** | 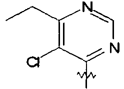 | 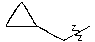 | |
| **5.4** | 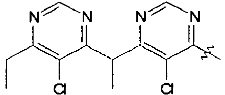 | 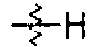 | **162** |
| **5.5** | 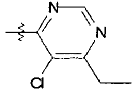 | 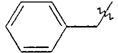 | |
| **6.6** | 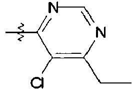 | 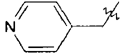 | |
| **5.7** | 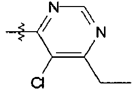 | 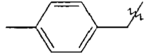 | |
| **5.8** | 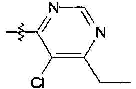 | 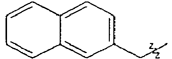 | |
| **5.9** | 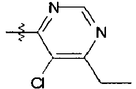 | 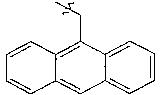 | |
| **5.10** | 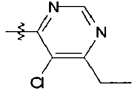 | 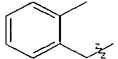 | |
| **5.11** | 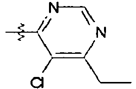 | 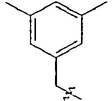 | |
| **5.12** | 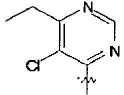 | 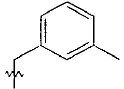 | |
| **5.13** | 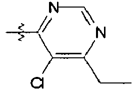 | 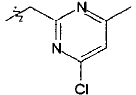 | |
| **5.14** | 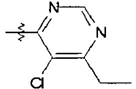 | 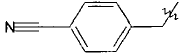 | |
| **5.16** | 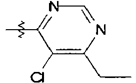 | 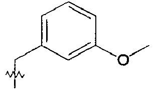 | |
| **5.16** | 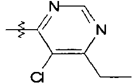 | 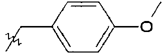 | |
| **5.17** | 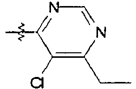 | 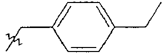 | |
| **5.18** | 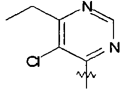 | 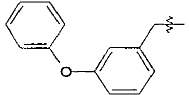 | |
| **6.19** | 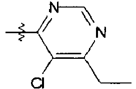 | 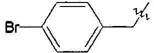 | |
| **6.20** | 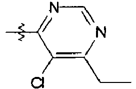 | 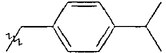 | |
| **5.21** | 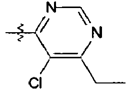 | 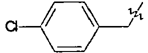 | |
| **5.22** | 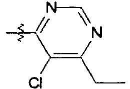 | 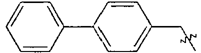 | |
| **5.23** | 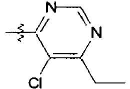 | 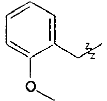 | |
| **5.24** | 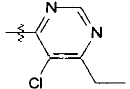 | 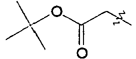 | |
| **5.25** | 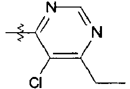 | 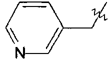 | |
| **5.26** | 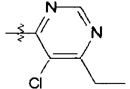 | 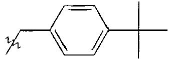 | |
| **5.27** | 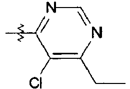 | 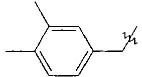 | |
| **5.28** | 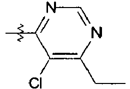 | 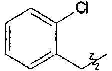 | |
| **5.29** | 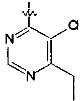 | 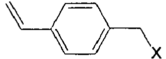 | |
| **5.30** | 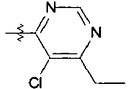 | 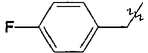 | |
| **5.31** | 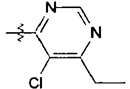 | 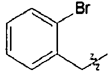 | |
| **5.32** | 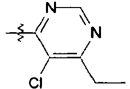 | 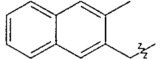 | |
| **5.33** | 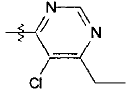 | 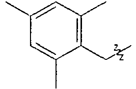 | |
| **5.34** | 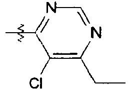 | 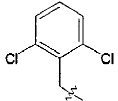 | |
| **5.35** | 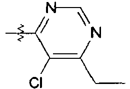 | 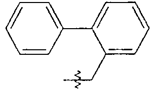 | |
| **5.36** | 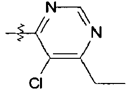 | 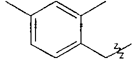 | |
| **5.37** | 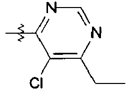 | 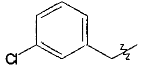 | |
| **5.38** | 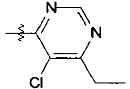 | 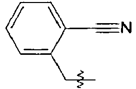 | |
| **5.39** | 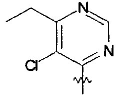 | 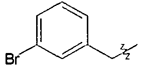 | |
| **5.40** | 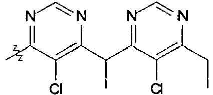 | 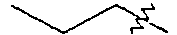 | |
| **5.41** | 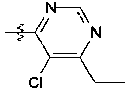 | 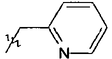 | |
| **5.42** | 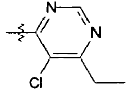 | 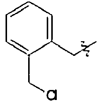 | |
| **5.43** | 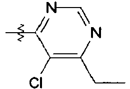 | 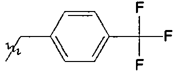 | |
| **5.44** | 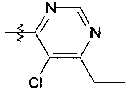 | 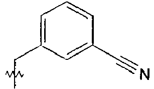 | |
| **5.45** | 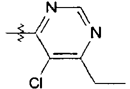 | 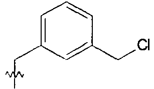 | |
| **5.46** | 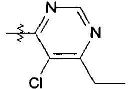 | 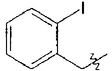 | |
| **5.47** | 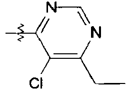 | 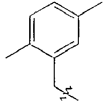 | |
| **5.48** | 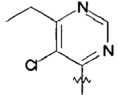 | 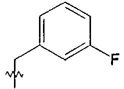 | |
| **5.49** | 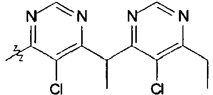 | 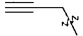 | |
| **5.50** | 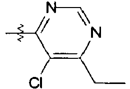 | 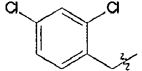 | |
| **5.51** | 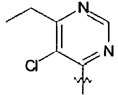 | 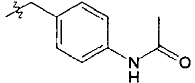 | |
| **5.52** | 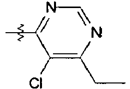 | 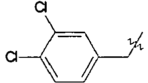 | |
| **5.53** | 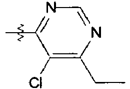 | 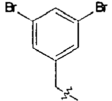 | |
| **5.54** | 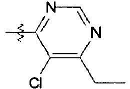 | 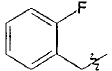 | |
| **5.55** | 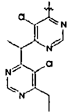 | 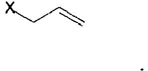 | |
| **5.56** | 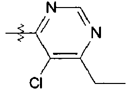 | 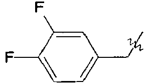 | |
| **5.57** | 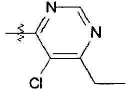 | 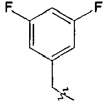 | |
| **5.58** | 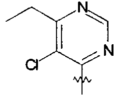 | 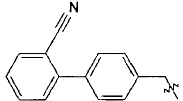 | |
| **5.59** | 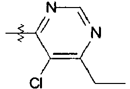 | 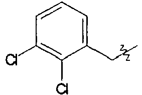 | |
| **5.60** | 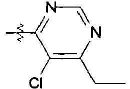 | 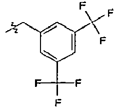 | |
| **5.61** | 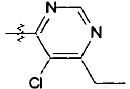 | 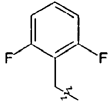 | |
| **5.62** | 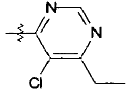 | 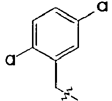 | |
| **5.63** | 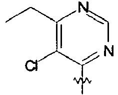 | 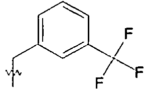 | |
| **5.64** | 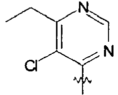 | 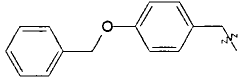 | |
| **5.65** | 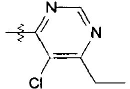 | 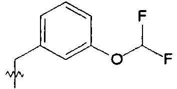 | |
| **5.66** | 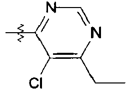 | 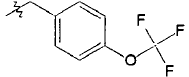 | |
| **5.67** | 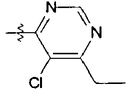 | 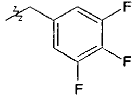 | |
| **5.68** | 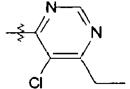 | 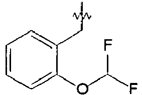 | |
| **5.69** | 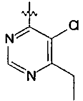 | 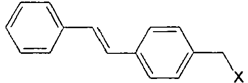 | |
| **5.70** | 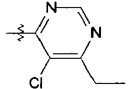 | 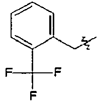 | |
| **5.71** | 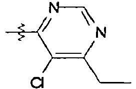 | 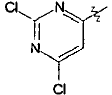 | |
| **5.72** | 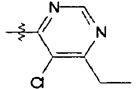 | 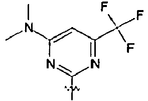 | |
| **5.73** | 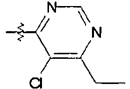 | 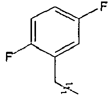 | |
| **5.74** | 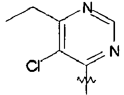 | 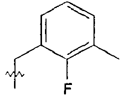 | |
| **5.75** | 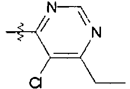 | 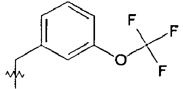 | |
| **5.76** | 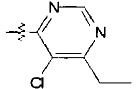 | 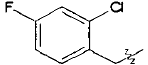 | |
| **5.77** | 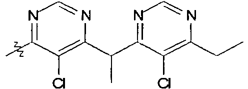 | 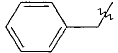 | |
| **5.78** | 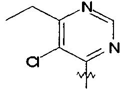 | 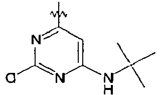 | |
| **5.79** | 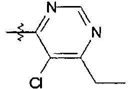 | 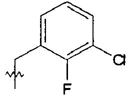 | |
| **5.80** | 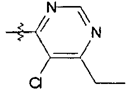 | 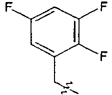 | |
| **5.81** | 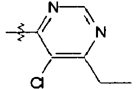 | 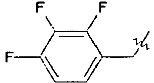 | |
| **6.82** | 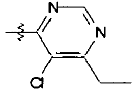 | 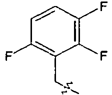 | |
| **5.83** | 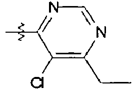 | 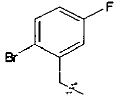 | |
| **5.84** | 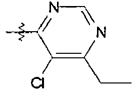 | 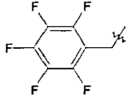 | |
| **5.85** | 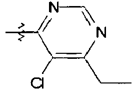 | 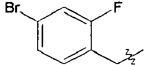 | **Oil** |
| **5.86** | 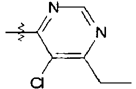 | 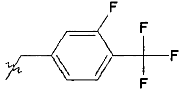 | |
| **5.87** | 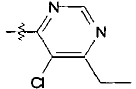 | 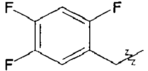 | |
| **5.88** | 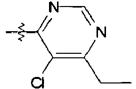 | 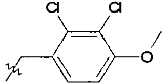 | |
| **5.89** | 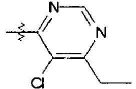 | 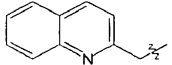 | |
| **5.90** | 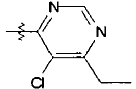 | 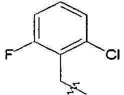 | |
| **6.91** | 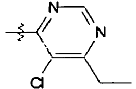 | 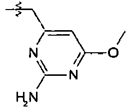 | |
| **5.92** | 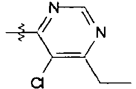 | 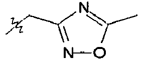 | |
| **5.93** | 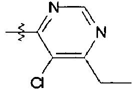 | 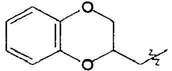 | |
| **5.94** | 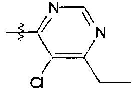 | 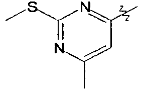 | |
| **5.95** | 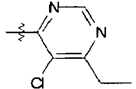 | 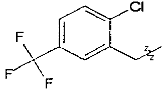 | |
| **5.96** | 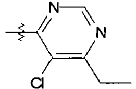 | 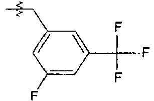 | |
| **5.97** | 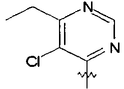 | 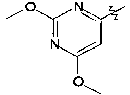 | |
| **5.98** | 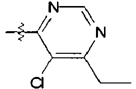 | 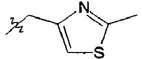 | |
| **5.99** | 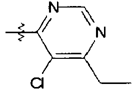 | 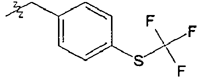 | |
| **5.100** | 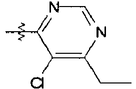 | 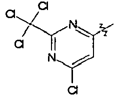 | |
| **5.101** | 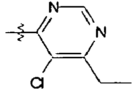 | 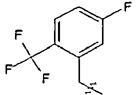 | |
| **5.102** | 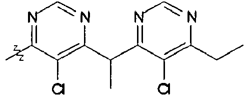 | 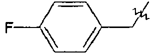 | |
| **5.103** | 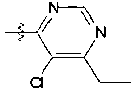 | 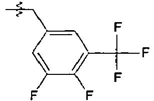 | |
| **5.104** | 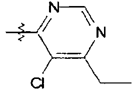 | 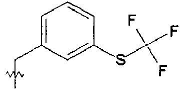 | |
| **5.105** | 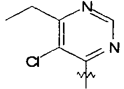 | 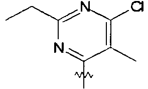 | |
| **5.106** | 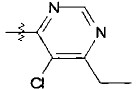 | 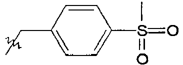 | |
| **5.107** | 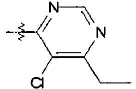 | 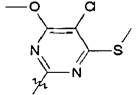 | |
| **5.108** | 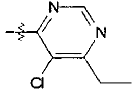 | 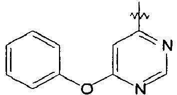 | |
| **5.109** | 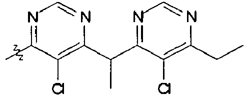 | 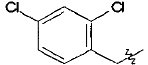 | |
| **5.110** | 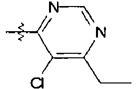 | 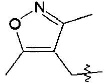 | |
| **5.111** | 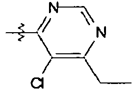 | 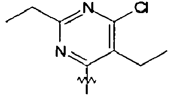 | |
| **5.112** | 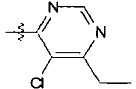 | 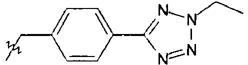 | |
| **5.113** | 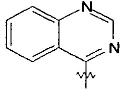 | 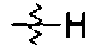 | |
| **5.114** | 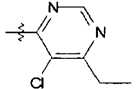 | 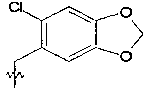 | |
| **5.115** | 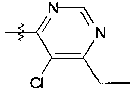 | 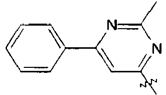 | |
| **5.116** | 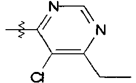 | 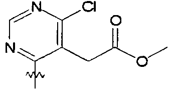 | |
| **5.117** | 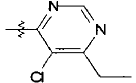 | 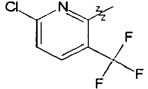 | |
| **5.118** | 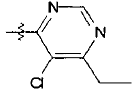 | 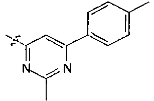 | |
| **5.119** | 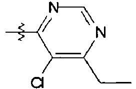 | 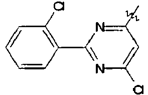 | |
| **5.120** | 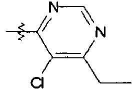 | 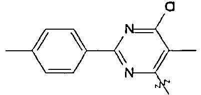 | |
| **6.121** | 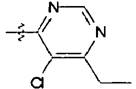 | 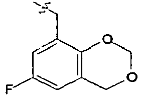 | |
| **6.122** | 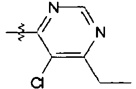 | 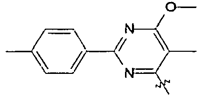 | |
| **5.123** | 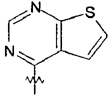 | 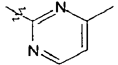 | |
| **5.124** | 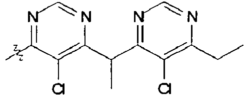 | 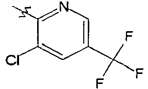 | |
| **5.125** | 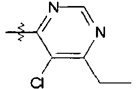 | 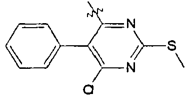 | |
| **5.126** | 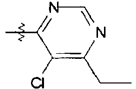 | 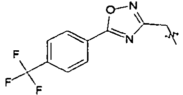 | |
| **5.127** | 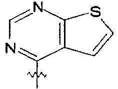 | 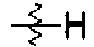 | |
| **5.128** | 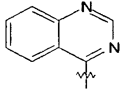 | 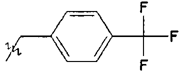 | |
| **5.129** | 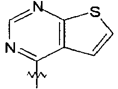 | 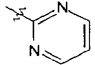 | |
| **5.130** | 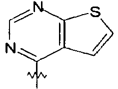 | 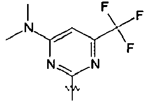 | |
| **5.131** | 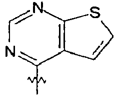 | 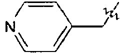 | |
| **5.132** | 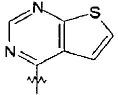 | 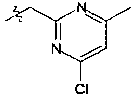 | |
| **5.133** | 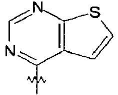 | 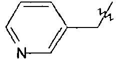 | |
| **5.134** | 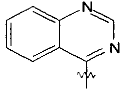 | 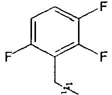 | |
| **5.136** | 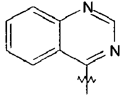 | 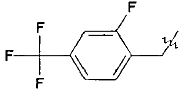 | |
| **5.136** | 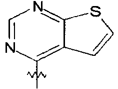 | 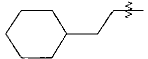 | |
| **5.137** | 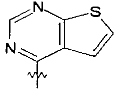 | 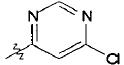 | |
| **5.138** | 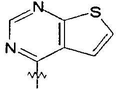 | 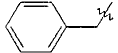 | **89** |
| **5.139** | 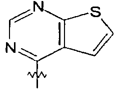 | 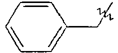 | |
| **5.140** | 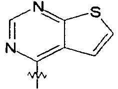 | 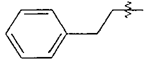 | |
| **5.141** | 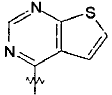 | 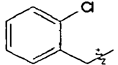 | |
| **5.142** | 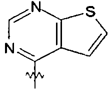 | 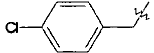 | **171** |
| **6.143** | 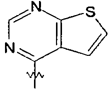 | 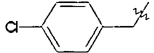 | |
| **5.144** | 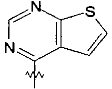 | 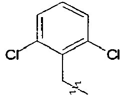 | |
| **5.145** | 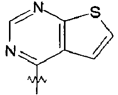 | 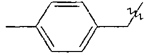 | **138** |
| **5.146** | 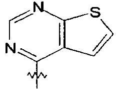 | 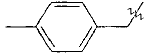 | |
| **6.147** | 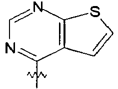 | 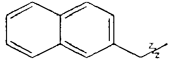 | |
| **5.148** | 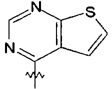 | 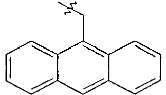 | |
| **5.149** | 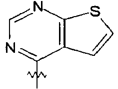 | 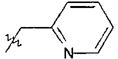 | |
| **5.150** | 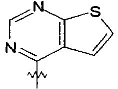 | 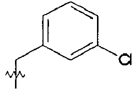 | |
| **5.151** | 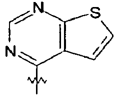 | 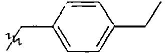 | |
| **5.152** | 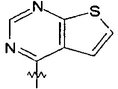 | 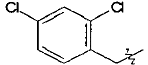 | |
| **5.153** | 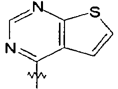 | 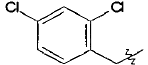 | |
| **5.154** | 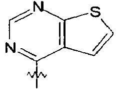 | 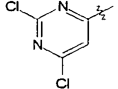 | |
| **5.155** | 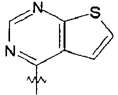 | 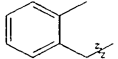 | |
| **5.156** | 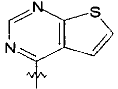 | 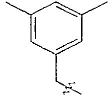 | |
| **5.157** | 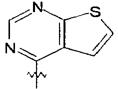 | 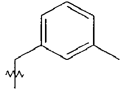 | |
| **5.158** | 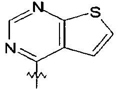 | 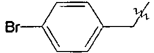 | |
| **5.159** | 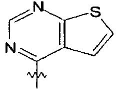 | 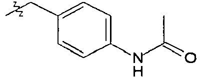 | |
| **5.160** | 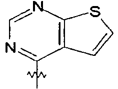 | 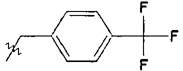 | |
| **5.161** | 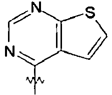 | 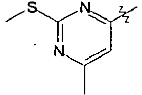 | |
| **6.162** | 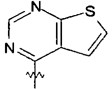 | 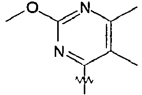 | |
| **5.163** | 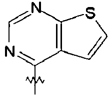 | 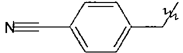 | |
| **5.164** | 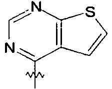 | 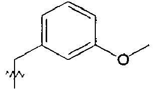 | |
| **5.165** | 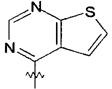 | 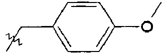 | |
| **6.166** | 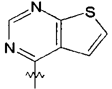 | 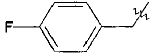 | |
| **5.167** | 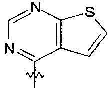 | 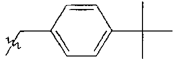 | |
| **5.168** | 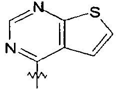 | 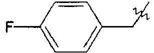 | |
| **5.169** | 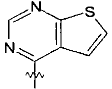 | 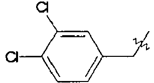 | |
| **5.170** | 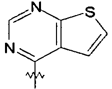 | 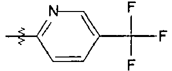 | **160** |
| **5.171** | 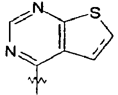 | 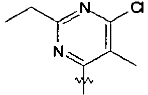 | |
| **5.172** | 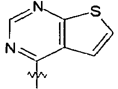 | 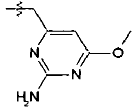 | |
| **5.173** | 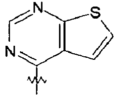 | 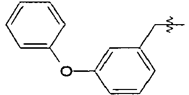 | |
| **6.174** | 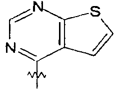 | 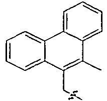 | |
| **5.175** | 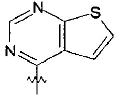 | 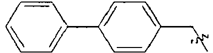 | |
| **5.176** | 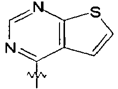 | 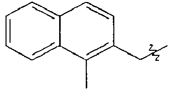 | |
| **5.177** | 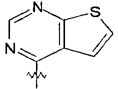 | 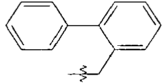 | |
| **5.178** | 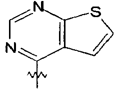 | 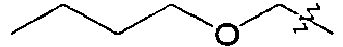 | |
| **6.179** | 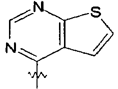 | 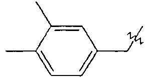 | |
| **5.180** | 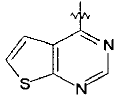 | 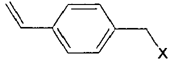 | |
| **5.181** | 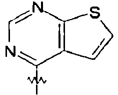 | 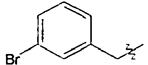 | |
| **5.182** | 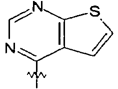 | 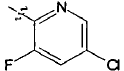 | |
| **5.183** | 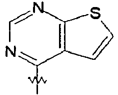 | 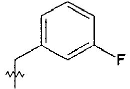 | |
| **5.184** | 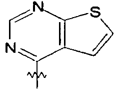 | 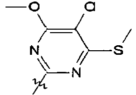 | |
| **5.185** | 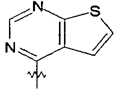 | 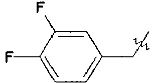 | |
| **5.186** | 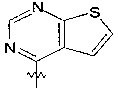 | 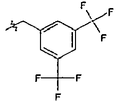 | |
| **5.187** | 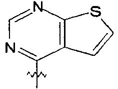 | 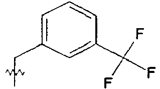 | |
| **6.188** | 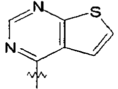 | 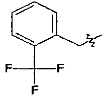 | |
| **5.189** | 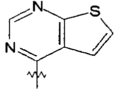 | 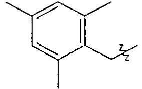 | |
| **5.190** | 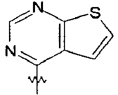 | 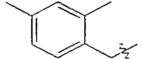 | |
| **6.191** | 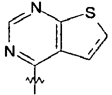 | 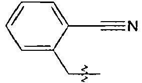 | |
| **5.192** | 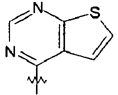 | 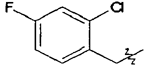 | |
| **5.193** | 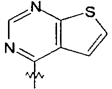 | 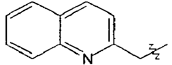 | |
| **5.194** | 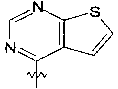 | 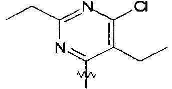 | |
| **5.195** | 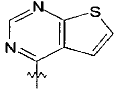 | 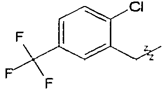 | |
| **6.196** | 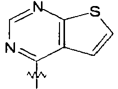 | 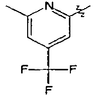 | **167** |
| **6.197** | 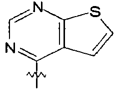 | 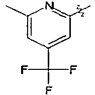 | |
| **6.198** | 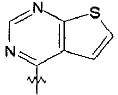 | 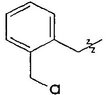 | |
| **5.199** | 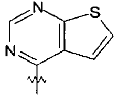 | 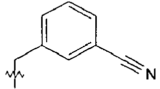 | |
| **5.200** | 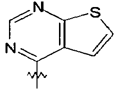 | 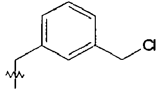 | |
| **5.201** | 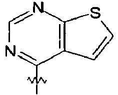 | 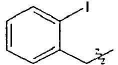 | |
| **5.202** | 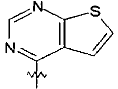 | 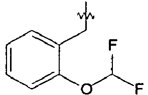 | |
| **5.203** | 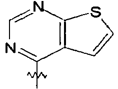 | 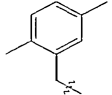 | |
| **5.204** | 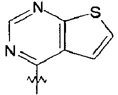 | 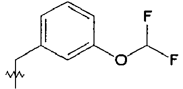 | |
| **5.205** | 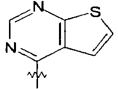 | 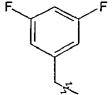 | |
| **5.206** | 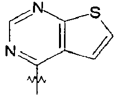 | 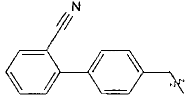 | |
| **5.207** | 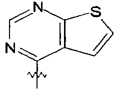 | 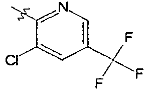 | **156** |
| **5.208** | 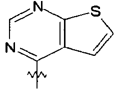 | 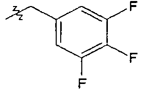 | |
| **5.209** | 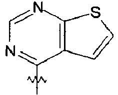 | 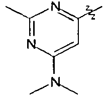 | |
| **5.210** | 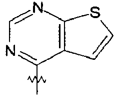 | 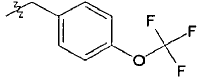 | |
| **5.211** | 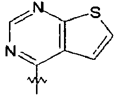 | 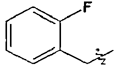 | |
| **5.212** | 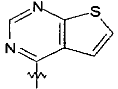 | 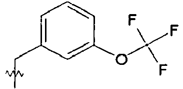 | |
| **5.213** | 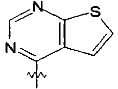 | 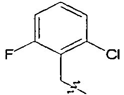 | |
| **5.214** | 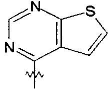 | 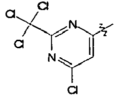 | |
| **5.215** | 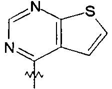 | 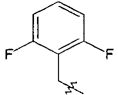 | |
| **6.216** | 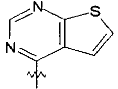 | 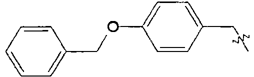 | |
| **5.217** | 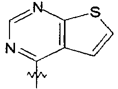 | 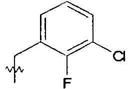 | |
| **5.218** | 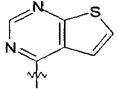 | 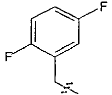 | |
| **5.219** | 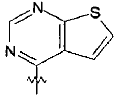 | 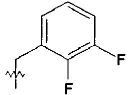 | |
| **5.220** | 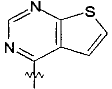 | 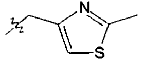 | |
| **5.221** | 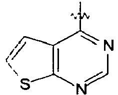 | 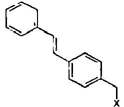 | |
| **5.222** | 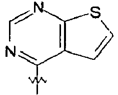 | 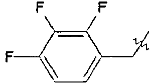 | **124** |
| **5.223** | 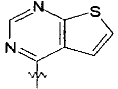 | 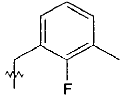 | |
| **5.224** | 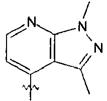 | 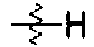 | **201** |
| **5.225** | 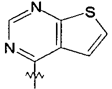 | 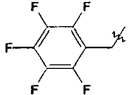 | |
| **5.226** | 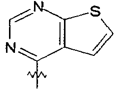 | 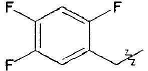 | **106** |
| **5.227** | 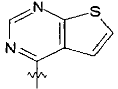 | 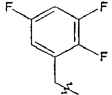 | |
| **5.228** | 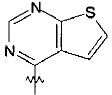 | 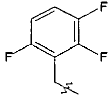 | |
| **5.229** | 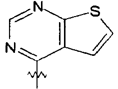 | 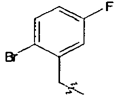 | |
| **5.230** | 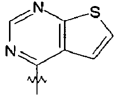 | 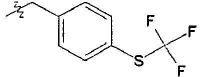 | |
| **5.231** | 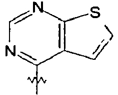 | 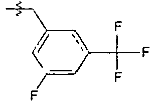 | |
| **5.232** | 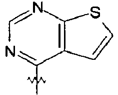 | 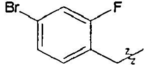 | |
| **5.233** | 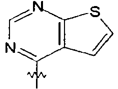 | 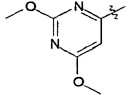 | |
| **5.234** | 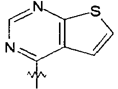 | 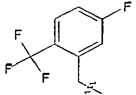 | |
| **5.236** | 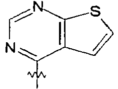 | 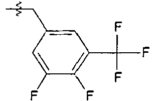 | |
| **5.236** | 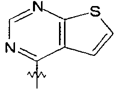 | 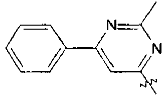 | |
| **5.237** | 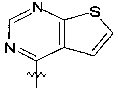 | 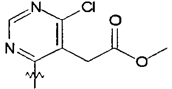 | |
| **5.238** | 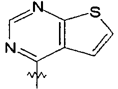 | 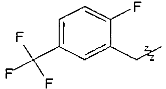 | |
| **5.239** | 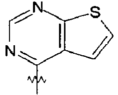 | 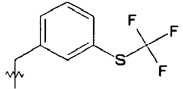 | |
| **5.240** | 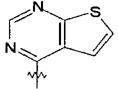 | 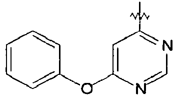 | |
| **6.241** | 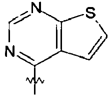 | 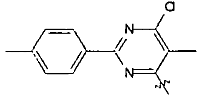 | |
| **5.242** | 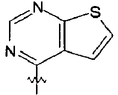 | 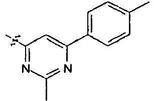 | |
| **5.243** | 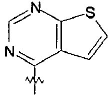 | 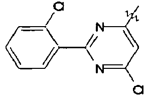 | |
| **5.244** | 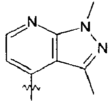 | 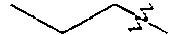 | |
| **5.245** | 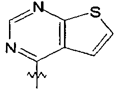 | 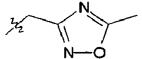 | |
| **6.246** | 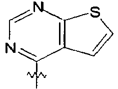 | 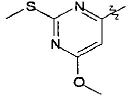 | |
| **5.247** | 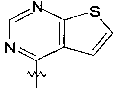 | 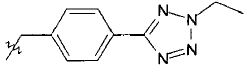 | |
| **5.248** | 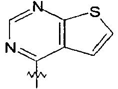 | 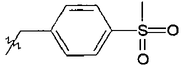 | |
| **5.249** | 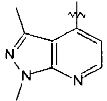 | 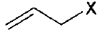 | **151** |
| **5.250** | 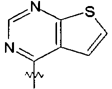 | 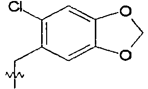 | |
| **5.251** | 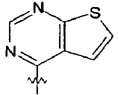 | 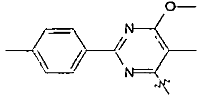 | |
| **5.252** | 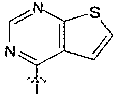 | 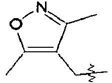 | |
| **5.263** | 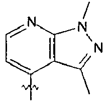 | 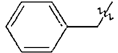 | |
| **6.264** | 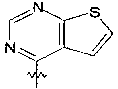 | 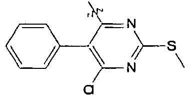 | |
| **5.255** | 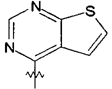 | 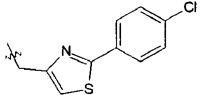 | |
| **5.256** | 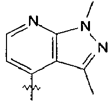 | 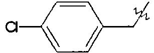 | |
| **5.257** | 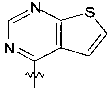 | 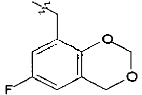 | |
| **5.258** | 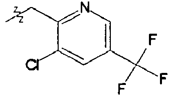 | 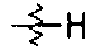 | |
| **5.259** | 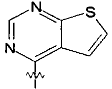 | 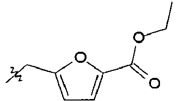 | |
| **5.260** | 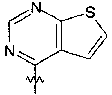 | 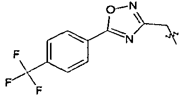 | |
| **5.261** | 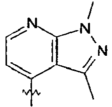 | 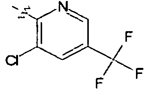 | **153** |
| **5.262** | 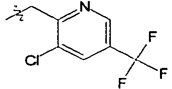 | 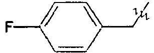 | |
| **5.263** | 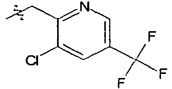 | 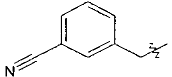 | |
| **5.264** | 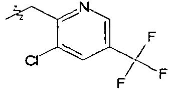 | 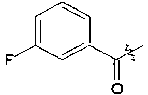 | |

### EXAMPLE 2

This Example illustrates the pesticidal/insecticidal properties of compounds of formula (I). Test against were performed as follows:
*Spodoptera littoralis* (Egyptian cotton leafworm):
Cotton leaf discs were placed on agar in a 24-well microtiter plate and sprayed with test solutions at an application rate of 200 ppm. After drying, the leaf discs were infested with 5 L₁ larvae. The samples were checked for mortality, repellent effect, feeding behaviour, and growth regulation 3 days after treatment (DAT). The following compounds gave at least 80% control of *Spodoptera littoralis*:
3.7, 3.9, 3.14, 3.20, 3.27, 3.32, 3.36, 5.29, 5.30, 5.211, 5.215, 5.232.
*Heliothis virescens* (Tobacco budworm):
Eggs (0-24 h old) were placed in 24-well microtiter plate on artificial diet and treated with test solutions at an application rate of 200 ppm by pipetting. After an incubation period of 4 days, samples were checked for egg mortality, larval mortality, and growth regulation. The following compounds gave at least 80% control of *Heliothis virescens*:
3.7, 3.8, 3.9, 3.12, 3.13, 3.14, 3.15, 3.20, 3.23, 3.27, 3.29, 3.30, 3.32, 3.36, 5.5, 5.14, 5.16, 5.21, 5.29, 5.30, 5.41, 5.73, 5.85, 5.87, 5.92, 5.128, 5.134, 5.135, 5.138, 5.139, 5.145, 5.146, 5.152, 5.153, 5.163, 5.211, 5.245.
*Plutella xylostella* (Diamond back moth):
24-well microtiter plate (MTP) with artificial diet was treated with test solutions at an application rate of 200 ppm by pipetting. After drying, the MTP's were infested with larvae (L2) (10-15 per well). After an incubation period of 5 days, samples were checked for larval mortality, antifeedant and growth regulation. The following compounds gave at least 80% control of *Plutella xylostella*:
3.6, 3.7, 3.9, 3.12, 3.20, 3.27, 3.29, 3.32, 3.35, 3.36, 3.38, 5.5, 5.7, 5.8, 5.10, 5.12, 5.14, 5.16, 5.17, 5.19, 5.21, 5.27, 5.28, 5.29, 5.30, 5.31, 5.33, 5.34, 5.36, 5.43, 5.44, 5.50, 5.54, 5.61, 5.66, 5.68, 5.70, 5.73, 5.74, 5.76, 5.79, 5.82, 5.85, 5.87, 5.88, 5.92, 5.128, 5.134, 5.135, 5.138, 5.139, 5.141, 5.142, 5.143, 5.145, 5.146, 5.147, 5.152, 5.153, 5.155, 5.160, 5.163, 5.164, 5.166, 5.168, 5.169, 5.179, 5.180, 5.185, 5.187, 5.188, 5.190, 5.192, 5.204, 5.210, 5.211, 5.213, 5.215, 5.217, 5.218, 5.219, 5.222, 5.223, 5.228, 5.232, 5.238, 5.245.
*Aedes aegypti* (Yellow fever mosquito):
10-15 Aedes larvae (L2) together with a nutrition mixture are placed in 96-well microtiter plates. Test solutions at an application rate of 2 ppm are pipetted into the wells. 2 days later, insects were checked for mortality and growth inhibition. The following compounds gave at least 80% control of *Aedes aegypti*:
3.6, 3.7, 3.8, 3.9, 3.12, 3.13, 3.14, 3.15, 3.18, 3.20, 3.23, 3.27, 3.28, 3.29, 3.30, 3.32, 3.35, 3.36, 3.38, 5.3, 5.5, 5.6, 5.7, 5.12, 5.15, 5.16, 5.17, 5.21, 5.25, 5.28, 5.29, 5.30, 5.36, 5.38, 5.41, 5.44, 5.48, 5.54, 5.61, 5.68, 5.70, 5.73, 5.76, 5.81, 5.82, 5.87, 5.88, 5.90, 5.92, 5.98, 5.112, 5.114, 5.128, 5.131, 5.134, 5.135, 5.138, 5.139, 5.141, 5.142, 5.143, 5.144, 5.145, 5.146, 5.147, 5.149, 5.150, 5.151, 5.152, 5.153, 5.155, 5.160, 5.163, 5.164, 5.166, 5.168, 5.179, 5.180, 5.181, 5.183, 5.185, 5.187, 5.188, 5.190, 5.191, 5.192, 5.193, 5.202, 5.204, 5.210, 5.211, 5.213, 5.215, 5.217, 5.218, 5.219, 5.222, 5.223, 5.227, 5.228, 5.232, 5.238, 5.245, 5.250, 5.255.

*Myzus persicae* (Green peach aphid): Sunflower leaf discs are placed on agar in a 24-well microtiter plate and sprayed with test solutions at an application rate of 200 ppm. After drying, the leaf discs are infested with an aphid population of mixed ages. After an incubation period of 6 DAT, samples are checked for mortality and special effects (e.g. phytotoxicity). The following compounds gave at least 80% control of *Myzus persicae:*
2.33, 3.14, 3.29, 3.32, 3.36, 5.7, 5.21, 5.29, 5.30, 5.43, 5.54, 5.76, 5.82, 5.114, 5.152, 5.153, 5.166, 5.168, 5.180, 5.190, 5.215, 5.218, 5.222, 5.232, 5.245.

*Tetranychus urticae* (Two-spotted spider mite): Bean leaf discs on agar in 24-well microtiter plates are sprayed with test solutions at an application rate of 200 ppm. After drying, the leaf discs are infested with mite populations of mixed ages. 8 days later, discs are checked for egg mortality, larval mortality, and adult mortality. The following compounds gave at least 80% control of *Tetranychus urticae:*
3.6, 3.7, 3.9, 3.14, 3.20, 3.27, 3.32, 3.36, 5.6, 5.16, 5.20, 5.22, 5.29, 5.43, 5.54, 5.66, 5.85, 5.86, 5.92, 5.99, 5.106, 5.128, 5.135, 5.138, 5.139, 5.145, 5.146, 5.147, 5.151, 5.160, 5.167, 5.170, 5.179, 5.180, 5.192, 5.210, 5.215, 5.217, 5.218, 5.228, 5.248, 5.260.

### EXAMPLE 3

This Example illustrates the fungicidal properties of compounds of formula (1).

The compounds were tested in a leaf disk assay, with methods described below. The test compounds were dissolved in DMSO and diluted into water to 200 ppm. In the case of the test on *Pythium ultimum,* they were dissolved in DMSO and diluted into water to 20 ppm.

*Erysiphe graminis f.sp. hordei* (barley powdery mildew): Barley leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.

*Erysiphe graminis f.sp. tritici* (wheat powdery mildew): Wheat leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.

*Puccinia recondita f.sp. tritici* (wheat brown rust): Wheat leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed nine days after inoculation as preventive fungicidal activity.

*Septoria nodorum* (wheat glume blotch): Wheat leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity. *Pyrenophora teres* (barley net blotch): Barley leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.

*Pyricularia oryzae* (rice blast): Rice leaf segments were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.

*Botrytis cinerea* (grey mould): Bean leaf disks were placed on agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.

*Phytophthora infestans* (late blight of potato on tomato): Tomato leaf disks were placed on water agar in a 24-well plate and sprayed with a solution of the test compound. After allowing to dry completely, for between 12 and 24 hours, the leaf disks were inoculated with a spore suspension of the fungus. After appropriate incubation the activity of a compound was assessed four days after inoculation as preventive fungicidal activity.

*Plasmopara viticola* (downy mildew of grapevine): Grapevine leaf disks were placed on agar in a 24-well plate and sprayed a solution of the test compound. After allowing to dry completely, for between 12 and 24 hours, the leaf disks were inoculated with a spore suspension of the fungus. After appropriate incubation the activity of a compound was assessed seven days after inoculation as preventive fungicidal activity.

*Septoria tritici* (leaf blotch): Conidia of the fungus from cryogenic storage were directly mixed into nutrient broth (PDB potato dextrose broth). After placing a (DMSO) solution of the test compounds into a microtiter plate (96-well format) the nutrient broth containing the fungal spores was added. The test plates were incubated at 24 C and the inhibition of growth was determined photometrically after 72 hrs.

*Botritis cinerea* (grey mould): Conidia of the fungus from cryogenic storage were directly mixed into nutrient broth (PDB potato dextrose broth). After placing a (DMSO) solution of the test compounds into a microtiter plate (96-well format) the nutrient broth containing the fungal spores was added. The test plates were incubated at 24 C and the inhibition of growth was determined photometrically after 72 hrs.

*Rhizoctonia solani* (foot rot and damping off ): Conidia of the fungus from cryogenic storage were directly mixed into nutrient broth (PDB potato dextrose broth). After placing a (DMSO) solution of the test compounds into a microtiter plate (96-well format) the nutrient broth containing the fungal spores was added. The test plates were incubated at 24 C and the inhibition of growth was determined photometrically after 72 hrs.

*Fusarium culmorum* (root rot): Conidia of the fungus from cryogenic storage were directly mixed into nutrient broth (PDB potato dextrose broth). After placing a (DMSO) solution of the test compounds into a microtiter plate (96-well format) the nutrient broth containing the fungal spores was added. The test plates were incubated at 24 C and the inhibition of growth was determined photometrically after 48 hrs.

*Pythium ultimum* (Damping off): Mycelial fragments of the fungus, prepared from a fresh liquid culture, were mixed into potato dextrose broth. A solution of the test compound in dimethyl sulphoxide was diluted with water to 20ppm then placed into a 96-well microtiter plate and the nutrient broth containing the fungal spores was added. The test plate was incubated at 24°C and the inhibition of growth was determined photometrically after 48 hours.

The following compounds (number of compound related to tables) gave at least 80% control of the following fungal infection at 200ppm: *Plasmopara viticola,* compounds 3.5; 3.17; 3.18; 3.20; 3.22; 3.23; 3.27; 3.29; 3.30; 3.32; 3.34; 3.35; 3.36; 3.38 ; 5.13; 5.16; 5.17; 5.19; 5.20; 5.23 ; 5.114; 5.117; 5.136; 5.138; 5.139; 5.141; 5.142; 5.143, 5.145; 5.146, 5.151; 5.154; 5.155; 5.157; 5.160; 5.166; 5.168; 5.169; 5.190; 5.191; 5.193; 5.215; 5.222; 5.228; 5.231; 5.238; 5.246; 5.250; 5.253; 5.255; 5.259 ;

*Phytophthora infestans,* compounds 3.3 ; 3.6 ; 3.25 ; 3.27 ; 3.29 ; 3.30; 5.2; 5.5; 5.6; 5.7 ; 5.10; 5.17; 5.19; 5.21; 5.24; 5.29; 5.41; 5.48; 5.51; 5.69; 5.81; 5.82; 5.91; 5.106; 5.110; 5.138, 5.139; 5.140; 5.141; 5.145, 5.146; 5.147; 5.150; 5.151; 5.157; 5.158; 5.159; 5.160; 5.163; 5.165; 5.166; 5.168; 5.177; 5.180; 5.181; 5.185; 5.189; 5.190; 5.199; 5.205; 5.210; 5.211; 5.219; 5.220; 5.227; 5.228; 5.232; 5.235; 5.237; 5.248; 5.255;

*Erysiphe graminis f.sp. tritici,* compounds 5.3; 5.4; 5.5; 5.7; 5.8; 5.9; 5.10; 5.12; 5.13; 5.14; 5.15; 5.16; 5.17; 5.18; 5.19; 5.20; 5.21; 5.22; 5.23; 5.24; 5.25; 5.26; 5.27; 5.28; 5.29; 5.30; 5.31; 5.33; 5.34; 5.35; 5.36; 5.37; 5.38; 5.39; 5.41; 5.42; 5.43; 5.44; 5.45; 5.46; 5.47; 5.48; 5.49; 5.51; 5.52; 5.53; 5.54; 5.56; 5.57; 5.58; 5.59; 5.61; 5.62; 5.64; 5.65; 5.66; 5.67; 5.68; 5.70; 5.71; 5.72; 5.73; 5.74; 5.75; 5.77; 5.79; 5.80; 5.81; 5.82 ;5.84 ; 5.87 ;5.88 ;5.89 ;5.90 ;5.91 ; 5.92 ; 5.93 ; 5.94 ;5.96 ; 5.97 ;5.98 ;5.99 ; 5.101; 5.102; 5.103; 5.106; 5.108; 5.112; 5.114; 5.121; 5.125; 5.126; 5.127; 5.130; 5.131; 5.132; 5.133; 5.138; 5.139; 5.140; 5.141; 5.142; 5.143; 5.144; 5.145; 5.146; 5.147; 5.149; 5.150; 5.151; 5.152; 5.153; 5.155; 5.156; 5.157; 5.158; 5.159; 5.160; 5.163; 5.164; 5.165; 5.166; 5.167; 5.168; 5.169; 5.170; 5.171; 5.172; 5.173; 5.175; 5.176; 5.178; 5.179; 5.181; 5.182; 5.183; 5.185; 5.186; 5.187; 5.188; 5.189; 5.190; 5.191; 5.193; 5.194 ; 5.195 ; 5.196 ; 5.197 ; 5.199 ; 5.200 ; 5.201 ; 5.202 ; 5.203 5.204 5.205 5.207 5.208 5.209 5.210 5.211 5.212 5.213 5.215 5.217 5.218 5.219 5.220 ; 5.222 ; 5.223 ; 5.225 ; 5.226 ;5.227 ; 5.228 ;5.230 ; 5.231 ; 5.232 ; 5.234 ;5.235 ; 5.237 ; 5.238 ;5.239 ;5.244 ;5.245 ; 5.247 ;5.248 ;5.249 ; 5.250 ; 5.251 ;5.255 ; 5.256 ; 5.257 ; 5.259 ;5.260 ; 5.261 ;

*Pyricularia oryzae,* compounds 3.9; 3.12; 3.13; 3.14; 3.15; 3.20; 3.23; 3.24; 3.27; 3.28; 3.29; 3.30; 3.31; 3.32; 3.34; 3.35; 3.36; 3.37; 3.38 ; 4.5 ; 4.9 ; 4.10 ; 5.14; 5.21; 5.43; 5.57; 5.86; 5.88 ; 5.128; 5.135; 5.140; 5.144; 5.149; 5.151; 5.158; 5.160; 5.163; 5.166; 5.168; 5.169; 5.173; 5.185; 5.199; 5.205; 5.215; 5.217; 5.218; 5.219; 5.234; 5.239; 5.245 ;

*Puccinia recondita f.sp. tritici,* compounds 3.1; 3.5; 3.7; 3.8; 3.10; 3.11; 3.12; 3.13; 3.14; 3.15; 3.18; 3.20; 3.22; 3.23; 3.24; 3.26; 3.27; 3.28; 3.29; 3.30; 3.31; 3.32; 3.34; 3.35; 3.36; 3.38; 5.3; 5.5; 5.6; 5.7; 5.8; 5.10; 5.11; 5.12; 5.14; 5.15; 5.16; 5.17; 5.18; 5.19; 5.20; 5.21; 5.22; 5.23; 5.27; 5.28; 5.29; 5.30; 5.31; 5.35; 5.36; 5.37; 5.38; 5.39; 5.41; 5.43; 5.44; 5.45; 5.46; 5.47; 5.48; 5.51; 5.54; 5.56; 5.57; 5.58; 5.59; 5.61; 5.64; 5.65; 5.66; 5.67; 5.68; 5.70; 5.73; 5.74; 5.76; 5.79; 5.80; 5.81; 5.82; 5.83; 5.84; 5.86; 5.87; 5.88; 5.89; 5.90; 5.91; 5.92; 5.93; 5.98; 5.99; 5.106; 5.110; 5.112; 5.114; 5.121; 5.126; 5.128; 5.134; 5.135; 5.138; 5.139; 5.140; 5.141; 5.142; 5.143; 5.144; 5.145; 5.146; 5.147; 5.149; 5.150; 5.151; 5.152; 5.153; 5.155; 5.156; 5.157; 5.158; 5.160; 5.163; 5.164; 5.166; 5.167; 5.168; 5.169; 5.170; 5.172; 5.173; 5.175; 5.178; 5.179; 5.181; 5.182; 5.183; 5.185; 5.187; 5.188; 5.189; 5.190; 5.191; 5.193; 5.196, 5.197; 5.199; 5.201; 5.202; 5.203; 5.204; 5.205; 5.206; 5.207; 5.208; 5.210; 5.211 ;5.212 ;5.213 ;5.215 ; 5.217 ; 5.218 ; 5.219 ; 5.220 ; 5.222 ; 5.223 ; 5.225 ; 5.226 ; 5.227 ;5.228 ;5.230 ;5.232 ;5.234 ;5.235 ; 5.238 ;5.239 ; 5.245 ;5.246 ; 5.247 ; 5.248 ; 5.250 ;5.254 ;5.255 ;5.256 ;5.257 ; 5.259 ;5.260 ;

The following compounds (number of compound related to tables) gave at least 60% control of the following fungal infection at 20ppm:

*Septoria tritici,* compounds 3.8 ; 3.14 ; 3.15 ; 3.35 ; 3.36 ;5.128; 5.134; 5.135 ; *Pyricularia oryzae,* compounds 3.6; 3.9; 3.11; 3.20; 3.27; 3.32; 3.34; 3.35; 3.36; 3.38; 5.30; 5.38; 5.61; 5.73; 5.76; 5.81; 5.82; 5.124; 5.128; 5.134; 5.135; 5.138, 5.139; 5.140; 5.141; 5.142; 5.143, 5.145; 5.146, 5.150; 5.152; 5.153; 5.155; 5.156; 5.157; 5.166; 5.168; 5.169; 5.170; 5.173; 5.178; 5.179; 5.181; 5.185; 5.187; 5.188; 5.192; 5.202; 5.203; 5.204; 5.207; 5.212; 5.215; 5.217; 5.218; 5.219; 5.222; 5.227; 5.228; 5.231; 5.232; 5.235; 5.236; 5.238; 5.256;

*Pythium ultimum,* compounds 5.3; 5.6; 5.41; 5.91; 5.98; 5.106; 5.128; 5.134; 5.135; 5.138, 5.139; 5.149; 5.165; 5.205; 5.218; 5.219; 5.222; 5.232; 5.248.

## Claims

1. Compounds of formula (I) 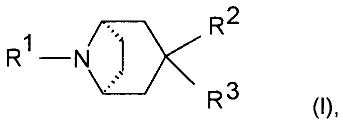 wherein
R¹ is pyrimidinyl quinazolinyl or thienopyrimidinyl, where these rings are optionally substituted by C₁₋₄alkyl, C₁₋₄alkoxy, C₁₋₄alkylthio, C₁₋₄haloalkyl, halogen, cyano or C₁₋₄alkylcarbonyl,
R³ together with R² form a radical of the formula =CH-R⁴, wherein R⁴ is phenyl, which is optionally substituted by C₁₋₄ -alkyl, C₁₋₄alkoxy or halogen, or R⁴ is allyloxymethyl or phenoxymethyl, which is optionally substituted by C₁₋₄alkyl, C₁₋₄alkoxy or halogen, or R⁴ is pyridyloxymethyl, which is optionally substituted by C₁₋₄alkyl, or R⁴ is pyrimidinyloxymethyl, which is optionally substituted by C₁₋₄alkyl, or R⁴ is C₁₋₆alkylcarbonyl, C₁₋₄alkyl-aminocarbonyl, benzylaminocarbonyl, phenylaminocarbonyl, which is optionally substituted by halogen, pyridylaminocarbonyl, which is optionally substituted by C₁₋₄alkyl, or pyridazineaminocarbonyl, which is optionally substituted by C₁₋₄alkyl or halogen, or
R³ together with R² form a radical of the formula =N-O-(CH₂)ₙ-R⁵, where n is 0 or 1, and R⁵ is C₁₋₄alkyl, C₁₋₄alkoxy, C₂₋₄alkenyl or C₂₋₄alkynyl, or R⁵ is phenyl, pyridyl or oxadiazoly, where these rings are optionally substituted by C₁₋₄alkyl, C₁₋₄alkoxy, C₁₋₄haloalkyl, halogen or -SO₂C₁₋₄alkyl,
or salts or N-oxides thereof.

2. Compounds according to claim 1, where R¹ is thienopyrimidinyl, which is optionally substituted by C₁₋₄alkyl, C₁₋₄alkoxy, C₁₋₄alkylthio, C₁₋₄haloalkyl, halogen, cyano or C₁₋₄alkylcarbonyl.

3. Compounds according to claim 1, wherein R⁴ is phenyl, which is optionally substituted by C₁₋₄ -alkyl, C₁₋₄alkoxy or halogen.

4. Compounds according to claim 1, wherein n is 1 and R⁵ is phenyl, which is optionally substituted by C₁₋₄alkyl, C₁₋₄alkoxy, C₁₋₄haloalkyl or halogen, or R⁵ is oxadiazolyl, which is optionally substituted by C₁₋₄alkyl.

5. An insecticidal, acaricidal, molluscicidal or nematicidal composition comprising an insecticidally, acaricidally, molluscicidally or nematicidally effective amount of a compound of the formula (I) according to claim 1.

6. A fungicidal composition comprising a fungicidally effective amount of the compound of the formula (I) according to claim 1.

7. A method of combating and controlling insects, acarines, nematodes or molluscs which comprises applying to a plant susceptible to attack by a pest an insecticidally, acaricidally, nematicidally or molluscicidally effective amount of a compound of formula (I) according to claim 1.

8. A method of combating and controlling phytopathogenic fungi which comprises applying to a plant, to a seed of a plant, to the locus of the plant or seed or to soil or to any other plant growth medium, a fungicidally effective amount of a compound of formula (I) according to claim 1.

9. Use of a compound of formula (I) according to claim 1 in the manufacture of a medicament for controlling pests on domestic animals and productive livestock.

## Patentansprüche

1. Verbindungen der Formel (I) 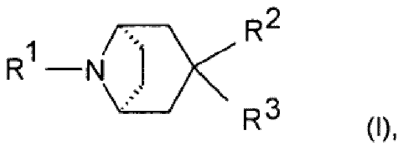 worin
R¹ Pyrimidinyl, Chinazolinyl oder Thienopyrimidinyl bedeutet, wobei diese Ringe gegebenenfalls durch C₁₋₄-Alkyl, C₁₋₄-Alkoxy, C₁₋₄-Alkylthio, C₁₋₄-Halogenalkyl, Halogen, Cyano oder C₁₋₄-Alkylcarbonyl substituiert sind,
R³ gemeinsam mit R² einen Rest der Formel =CH-R⁴ bedeutet, wobei R⁴ Phenyl, das gegebenenfalls durch C₁₋₄-Alkyl, C₁₋₄-Alkoxy oder Halogen substituiert ist, bedeutet, oder R⁴ Allyloxymethyl oder Phenoxymethyl, das gegebenenfalls durch C₁₋₄-Alkyl, C₁₋₄-Alkoxy oder Halogen substituiert ist, bedeutet, oder R⁴ Pyridyloxymethyl, das gegebenenfalls durch C₁₋₄-Alkyl substituiert ist, bedeutet, oder R⁴ Pyrimidinyloxymethyl, das gegebenenfalls durch C₁₋₄-Alkyl substituiert ist, bedeutet, oder R⁴ C₁₋₆-Alkylcarbonyl, C₁₋₄-Alkylaminocarbonyl, Benzylaminocarbonyl, Phenylaminocarbonyl, das gegebenenfalls durch Halogen substituiert ist, Pyridylaminocarbonyl, das gegebenenfalls durch C₁₋₄-Alkyl substituiert ist, oder Pyridazinaminocarbonyl, das gegebenenfalls durch C₁₋₄-Alkyl oder Halogen substituiert ist, bedeutet, oder
R³ gemeinsam mit R² einen Rest der Formel =N-O-(CH₂)ₙ-R⁵ bildet, wobei n 0 oder 1 bedeutet und R⁵ C₁₋₄-Alkyl, C₁₋₄-Alkoxy, C₂₋₄-Alkenyl oder C₂₋₄-Alkinyl bedeutet, oder R⁵ Phenyl, Pyridyl oder Oxadiazolyl bedeutet, wobei diese Ringe gegebenenfalls durch C₁₋₄-Alkyl, C₁₋₄-Alkoxy, C₁₋₄-Halogenalkyl, Halogen oder -SO₂C₁₋₄-Alkyl substituiert sind,
oder Salze oder N-Oxide davon.

2. Verbindungen nach Anspruch 1, wobei R¹ Thienopyrimidinyl, das gegebenenfalls durch C₁₋₄-Alkyl, C₁₋₄-Alkoxy, C₁₋₄-Alkylthio, C₁₋₄-Halogenalkyl, Halogen, Cyano oder C₁₋₄-Alkylcarbonyl substituiert ist, bedeutet.

3. Verbindungen nach Anspruch 1, wobei R⁴ Phenyl, das gegebenenfalls durch C₁₋₄-Alkyl, C₁₋₄-Alkoxy oder Halogen substituiert ist, bedeutet.

4. Verbindungen nach Anspruch 1, worin n 1 bedeutet und R⁵ Phenyl, das gegebenenfalls durch C₁₋₄-Alkyl, C₁₋₄-Alkoxy, C₁₋₄-Halogenalkyl oder Halogen substituiert ist, bedeutet, oder R⁵ Oxadiazolyl, das gegebenenfalls durch C₁₋₄-Alkyl substituiert ist, bedeutet.

5. Insektizide, akarizide, molluskizide oder nematizide Zusammensetzung, umfassend eine insektizid, akarizid, molluskizid oder nematizid wirksame Menge einer Verbindung der Formel (I) nach Anspruch 1.

6. Fungizide Zusammensetzung, umfassend eine fungizid wirksame Menge der Verbindung der Formel (I) nach Anspruch 1.

7. Verfahren zum Bekämpfen und Kontrollieren von Insekten, Acarina, Nematoden oder Mollusken, bei dem man eine insektizid, akarizid, nematizid oder molluskizid wirksame Menge einer Verbindung der Formel (I) nach Anspruch 1 auf eine Pflanze, die von einem Schädling befallen werden kann, appliziert.

8. Verfahren zum Bekämpfen und Kontrollieren von phytopathogenen Pilzen, bei dem man auf eine Pflanze, auf einen Samen einer Pflanze, auf den Ort der Pflanze oder des Samens oder auf Boden oder auf ein beliebiges sonstiges Pflanzensubstrat eine fungizid wirksame Menge einer Verbindung der Formel (I) nach Anspruch 1 appliziert.

9. Verwendung einer Verbindung der Formel (I) nach Anspruch 1, in der Herstellung eines Arzneimittels für die Bekämpfung von Schädlingen an Haustieren und Nutztieren.

## Revendications

1. Composés de formule (I) 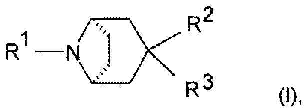 dans laquelle
R¹ est pyrimidinyle, quinazolinyle ou thiénopyrimidinyle, où ces cycles sont éventuellement substitués par C₁₋₄alkyle, C₁₋₄alcoxy, C₁₋₄alkylthio, C₁₋₄halogénoalkyle, halogène, cyano ou C₁₋₄alkylcarbonyle ;
R³ conjointement avec R² forme un radical de formule =CH-R⁴, où R⁴ est phényle, qui est éventuellement substitué par C₁₋₄-alkyle, C₁₋₄alcoxy ou halogène, ou R⁴ est allyloxyméthyle ou phénoxyméthyle, qui est éventuellement substitué par C₁₋₄alkyle, C₁₋₄alcoxy ou halogène, ou R⁴ est pyridyloxyméthyle, qui est éventuellement substitué par C₁₋₄alkyle, ou R⁴ est pyrimidinyloxyméthyle, qui est éventuellement substitué par C₁₋₄alkyle, ou R⁴ est C₁₋₆alkylcarbonyle, C₁₋₄alkyl-aminocarbonyle, benzylaminocarbonyle, phénylamino-carbonyle, qui est éventuellement substitué par halogène, pyridylaminocarbonyle, qui est éventuellement substitué par C₁₋₄alkyle, ou pyridazineaminocarbonyle, qui est éventuellement substitué par C₁₋₄alkyle ou halogène ; ou
R³ conjointement avec R² forme un radical de formule =N-O-(CH₂)ₙ-R⁵, où n vaut 0 ou 1, et R⁵ est C₁₋₄alkyle, C_{1- 4}alcoxy, C₂₋₄alcényle ou C₂₋₄alcynyle, ou R⁵ est phényle, pyridyle ou oxadiazolyle, où ces cycles sont éventuellement substitués par C₁₋₄alkyle, C₁₋₄alcoxy, C₁₋₄halogénoalkyle, halogène ou -SO₂C₁₋₄alkyle ;
ou les sels ou N-oxydes de ceux-ci.

2. Composés selon la revendication 1, dans lesquels R¹ est thiénopyrimidinyle, qui est éventuellement substitué par C₁₋₄alkyle, C₁₋₄alcoxy, C₁₋₄alkylthio, C₁₋₄halogénoalkyle, halogène, cyano ou C₁₋₄alkylcarbonyle.

3. Composés selon la revendication 1, dans lesquels R⁴ est phényle, qui est éventuellement substitué par C₁₋₄-alkyle, C₁₋₄alcoxy ou halogène.

4. Composés selon la revendication 1, dans lesquels n vaut 1 et R⁵ est phényle, qui est éventuellement substitué par C₁₋₄alkyle, C₁₋₄alcoxy, C₁₋₄halogénoalkyle ou halogène, ou R⁵ est oxadiazolyle, qui est éventuellement substitué par C₁₋₄alkyle.

5. Composition insecticide, acaricide, molluscicide ou nématicide comprenant une quantité efficace sur le plan insecticide, acaricide, molluscicide ou nématicide d'un composé de formule (I) selon la revendication 1.

6. Composition fongicide comprenant une quantité efficace sur le plan fongicide d'un composé de formule (I) selon la revendication 1.

7. Méthode de lutte et de contrôle d'insectes, d'acariens, de nématodes ou de mollusques, comprenant l'application à une plante susceptible d'être attaquée par un organisme nuisible, d'une quantité efficace sur le plan insecticide, acaricide, nématicide ou molluscicide d'un composé de formule (I) selon la revendication 1.

8. Méthode de lutte et de contrôle de champignons phytopathogènes, comprenant l'application à une plante, à des semences d'une plante, au lieu où se développent la plante ou les semences, ou au sol ou à tout autre milieu de croissance de la plante, d'une quantité efficace sur le plan fongicide d'un composé de formule (I) selon la revendication 1.

9. Utilisation d'un composé de formule (I) selon la revendication 1, dans l'élaboration d'un médicament destiné au contrôle d'organismes nuisibles sur des animaux domestiques et des cheptels de production.
